(19) Europäisches Patentamt
European Patent Office
Office européen des brevets

(11) EP 0 600 318 B1

(12) **EUROPÄISCHE PATENTSCHRIFT**

(45) Veröffentlichungstag und Bekanntmachung des Hinweises auf die Patenterteilung:
**08.04.1998 Patentblatt 1998/15**

(51) Int. Cl.$^6$: **C07C 49/813**, C07C 205/45, C07C 49/84, C07C 49/86, C07C 45/46, C07C 45/63

(21) Anmeldenummer: **93118602.7**

(22) Anmeldetag: **18.11.1993**

(54) **Neue asymmetrische, halogenierte Benzophenone und Verfahren zu ihrer Herstellung**
Asymmetric, halogenated benzophenones and process for their preparation
Benzophénones asymétriques, halogénées et procédé de préparation

(84) Benannte Vertragsstaaten:
**BE CH DE ES FR GB IT LI NL**

(30) Priorität: **29.11.1992 DE 4240022**

(43) Veröffentlichungstag der Anmeldung:
**08.06.1994 Patentblatt 1994/23**

(73) Patentinhaber: **Clariant GmbH**
**65929 Frankfurt am Main (DE)**

(72) Erfinder:
- **Pfirmann, Ralf, Dr.**
  **D-64347 Griesheim (DE)**
- **Rapp, Jochen, Dr.**
  **D-60322 Frankfurt am Main (DE)**
- **Forstinger, Klaus, Dr.**
  **D-65451 Kelsterbach (DE)**
- **Papenfuhs, Theodor, Dr.**
  **D-60433 Frankfurt am Main (DE)**

(56) Entgegenhaltungen:
**EP-A- 0 084 742**
**EP-A- 0 101 760**
**EP-A- 0 151 529**
**DE-A- 2 014 514**
**DE-C- 525 187**
**FR-A- 2 254 331**
**FR-A- 2 346 350**
**FR-A- 2 397 412**
**FR-A- 2 534 905**
**GB-A- 923 115**
**US-A- 2 879 296**
**US-A- 4 120 687**
**US-A- 5 210 313**

- **DATABASE WPI Week 9044, Derwent Publications Ltd., London, GB; AN 90-332346 & JP-A-2 240 042 (IHARA CHEM IND) 25. September 1990**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 600 318 B1

**Beschreibung**

Die vorliegende Erfindung betrifft neue asymmetrische, halogenierte Benzophenone, die neue Zwischenprodukte für die Herstellung von Pharmazeutika, Pflanzenschutzmitteln und Flüssigkristallen darstellen, sowie ein Verfahren zu ihrer Herstellung. Die neuen Verbindungen können nach einem neuartigen Verfahren in Halogenbenzoesäuren und gegebenenfalls gleichzeitig in Phenole umgewandelt werden (Patentanmeldung P 42 40 020.1 vom gleichen Anmeldedatum). Beide Umwandlungsprodukte stellen ihrerseits wertvolle Zwischenprodukte für die vorstehend angegebenen Anwendungen dar.

Gegenstand der vorliegenden Erfindung sind asymmetrische halogenierte Benzophenone der allgemeinen Formel

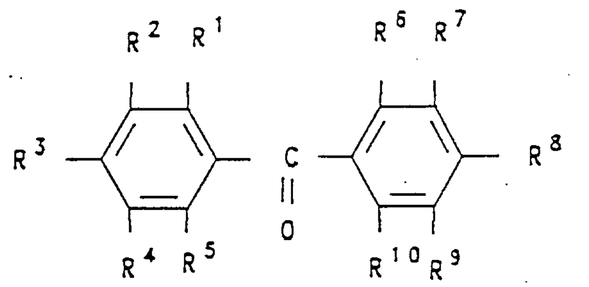

(1)

worin $R^1$, $R^3$ für Chlor, $R^4$ für Fluor oder $R^1$ für Chlor oder Fluor, $R^3$, $R^4$ für Fluor oder $R^1$ für Chlor, $R^3$ für Fluor, $R^4$ für Wasserstoff und $R^2$, $R^5$, $R^6$, $R^7$, $R^8$, $R^9$ und $R^{10}$ für Wasserstoff oder $R^1$ für Chor, $R^3$, $R^4$ und $R^8$ für Fluor und $R^2$, $R^5$, $R^6$, $R^7$, $R^9$ und $R^{10}$ für Wasserstoff oder $R^1$, $R^2$, $R^3$ für Chlor, $R^4$ für Chlor oder Wasserstoff, $R^8$ für Fluor und $R^5$, $R^6$, $R^7$, $R^9$ und $R^{10}$ für Wasserstoff oder $R^1$, $R^3$, $R^4$ für Chlor, $R^6$ für Fluor und $R^2$, $R^5$, $R^7$, $R^8$, $R^9$ und $R^{10}$ für Wasserstoff oder $R^1$, $R^6$ für Chlor, $R^2$, $R^3$, $R^4$ für Fluor, $R^5$, $R^7$, $R^8$, $R^9$ und $R^{10}$ für Wasserstoff oder $R^1$, $R^2$, $R^3$, $R^4$ für Fluor, $R^7$ für Chlor oder Wasserstoff, $R^8$ für Chlor, und $R^5$, $R^6$, $R^9$ und $R^{10}$ für Wasserstoff oder $R^2$, $R^3$ für Chlor, $R^8$ für Fluor und $R^1$, $R^4$, $R^5$, $R^6$, $R^7$, $R^9$ und $R^{10}$ für Wasserstoff oder $R^1$, $R^3$ für Chlor, $R^4$ für Fluor, $R^8$ für Methyl und $R^2$, $R^5$, $R^6$, $R^7$, $R^9$ und $R^{10}$ für Wasserstoff oder $R^3$ für Fluor, $R^8$ für Methoxy, $R^1$, $R^2$, $R^4$, $R^5$, $R^6$, $R^7$, $R^9$ und $R^{10}$ für Wasserstoff oder $R^2$, $R^3$ für Fluor, $R^1$ und $R^4$ bis $R^{10}$ für Wasserstoff stehen.

Gegenstand der Erfindung ist ferner ein Verfahren zur Herstellung von asymmetrischen halogenierten Benzophenonen der allgemeinen Formel

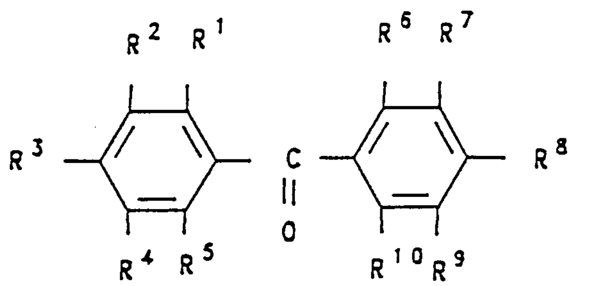

(1)

worin $R^1$, $R^3$ für Chlor, $R^4$ für Fluor oder $R^1$ für Chlor oder Fluor, $R^3$, $R^4$ für Fluor oder $R^1$ für Chlor, $R^3$ für Fluor, $R^4$ für Wasserstoff und $R^2$, $R^5$, $R^6$, $R^7$, $R^8$, $R^9$ und $R^{10}$ für Wasserstoff oder $R^1$ für Chlor, $R^3$, $R^4$ und $R^8$ für Fluor und $R^2$, $R^5$, $R^6$, $R^7$, $R^9$ und $R^{10}$ für Wasserstoff oder $R^1$, $R^2$, $R^3$ für Chlor, $R^4$ für Chlor oder Wasserstoff, $R^8$ für Fluor und $R^5$, $R^6$, $R^7$, $R^9$ und $R^{10}$ für Wasserstoff oder $R^1$, $R^3$, $R^4$ für Chlor, $R^6$ für Fluor und $R^2$, $R^5$, $R^7$, $R^8$, $R^9$ und $R^{10}$ für Wasserstoff oder $R^1$, $R^6$ für Chlor, $R^2$, $R^3$, $R^4$ für Fluor, $R^5$, $R^7$, $R^8$, $R^9$ und $R^{10}$ für Wasserstoff oder $R^1$, $R^2$, $R^3$, $R^4$ für Fluor, $R^7$ für Chlor oder Wasserstoff, $R^8$ für Chlor, und $R^5$, $R^6$, $R^9$ und $R^{10}$ für Wasserstoff oder $R^2$, $R^3$ für Chlor, $R^8$ für Fluor und $R^1$, $R^4$, $R^5$, $R^6$, $R^7$, $R^9$ und $R^{10}$ für Wasserstoff oder $R^1$, $R^3$ für Chlor, $R^4$ für Fluor, $R^8$ für Methyl und $R^2$, $R^5$, $R^6$, $R^7$, $R^9$ und $R^{10}$ für Wasserstoff oder $R^3$ für Fluor, $R^8$ für Methoxy, $R^1$, $R^2$, $R^4$, $R^5$, $R^6$, $R^7$, $R^9$ und $R^{10}$ für Wasserstoff oder $R^2$, $R^3$ für Fluor, $R^1$ und $R^4$ bis $R^{10}$ für Wasserstoff stehen. Es ist dadurch gekennzeichnet, daß man 1 Mol eines halogenierten Benzols der allgemeinen Formel (2)

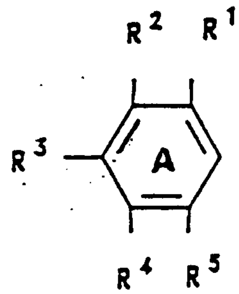

(2)

in welcher $R^1$, $R^2$, $R^3$, $R^4$, $R^5$ die vorstehend genannten Bedeutungen besitzen mit 1 bis 5 Mol vorzugsweise 1,05 bis 2 Mol eines Benzoylhalogenids der allgemeinen Formel (3)

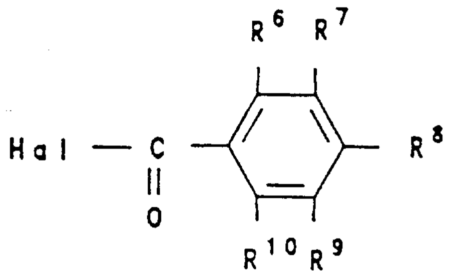

(3)

in welcher Hal ein Fluor-, Chlor- oder Bromatom und $R^6$, $R^7$, $R^8$, $R^9$ und $R^{10}$ die vorstehend genannten Bedeutungen haben, in Gegenwart eines Acylierungskatalysators bei Temperaturen von 0 °C bis 230 °C vorzugsweise 70 bis 150 °C in Abwesenheit oder in Gegenwart eines gegenüber den Reaktionsteilnehmern inerten organischen Lösungsmittels acyliert und ggfs. die so erhaltenen Benzophenone der genannten Formel (1), sofern mindestens einer der Substituenten $R^1$ - $R^{10}$ ein Chloratom darstellt, ggfs. nach ihrer Zwischenisolierung mit 1 bis 2,5 Mol insbesondere 1,01 bis 1,5 Mol, bevorzugt 1,05 bis 1,2 Mol eines Kalium-, Rubidium- oder Cäsiumfluorids oder Mischungen davon pro auszutauschendem Chloratom bei Temperaturen von 120 bis 280 °C vorzugsweise 160 bis 230 °C in Abwesenheit oder in Gegenwart eines Phasentransferkatalysators und in Abwesenheit oder in Gegenwart eines dipolar aprotischen Lösemittels umsetzt.

Als Acylierungskatalysator kann eine Lewis-Säure , wie beispielsweise Aluminiumchlorid, Aluminiumbromid, Antimon(V)-chlorid, Eisen(III)-chlorid, Eisen(II)-chlorid, Titan(IV)-chlorid, Bortrifluorid, Zinn(IV)-chlorid, Wismut(III)-chlorid, Zinkchlorid oder Quecksilber(II)-chlorid, oder eine Brönsted-Säure, wie beispielsweise Fluorwasserstoff, Schwefelsäure, Polyphosphorsäure, p-Toluolsulfonsäure, Fluoralkansulfonsäuren, wie beispielsweise Trifluormethansulfonsäure oder Hexafluorpropansulfonsäure, verwendet werden. Bevorzugt wird Eisen(III)-chlorid, Aluminiumchlorid und/oder Aluminiumtribromid, besonders bevorzugt Aluminiumtrichlorid eingesetzt.

Die Acylierungskatalysatoren werden in Mengen von 100 bis 500 Molprozent, vorzugsweise von 200 bis 300 Molprozent, bezogen auf eingesetztes Benzoylhalogenid, eingesetzt.

Zu den weiter oben genannten geeigneten Acylierungskatalysatoren stellen, aus ökologischer Sicht sehr vorteilhaft, die neuen Acylierungskatalysatoren vom Typ der metalldotierten Tonerdemineralien, wie beispielsweise metalldotierter Montmorillonit, eine wertvolle Alternative dar. Solche Produkte sind unter dem Namen Envirocats ® bekannt geworden. Beschrieben werden sie beispielsweise unter CAS-Nr. 1318-93-0, 76 46-85-7 und 7705-08-0.

Sie besitzen teilweise erhebliche Vorteile bei Benzoylierungen, so die Mengenreduzierung gegenüber den klassischen Acylierungskatalysatoren, die einfache Abtrennung aus der Reaktionsmischung durch Filtration, die Recyclisierbarkeit, bessere Handhabung durch Reaktionsträgheit gegenüber Wasser, geringe Korrosions- und Reizwirkung, teilweise höhere Selektivität sowie unproblematische Entsorgung des festen, verbrauchten Materials. Solche Materialien werden in Mengen von 2 Massenprozent bis 100 Massenprozent, bevorzugt zwischen 10 Massenprozent und 30 Massenprozent, bezogen auf das verwendete Benzoylhalogenid, eingesetzt. Verwendet man metalldotierte Tonerdeminderalien ("Envirocats"), so arbeitet man bevorzugt bei Temperaturen von 140 °C bis 220 °C und daher, in Abhängigkeit vom Siedepunkt der Reaktionsverbindungen, bei Überdruck. In diesem Fall sind die Umsetzungsgeschwindigkeiten geringer und Reaktionszeiten zwischen etwa 5 h und etwa 48 h möglich. Bei Verwendung herkömmlicher Katalysatoren liegen diese zwischen etwa 3 h und etwa 18 h.

Das Benzoylhalogenid wird üblicherweise in einer Menge von 1 bis 5 Mol, vorzugsweise 1,05 bis 2 mol, bezogen

auf das eingesetzte halogenierte Benzol verwendet.

Als gegenüber den Reaktionsteilnehmern inerte organische Lösungsmittel können bei der Acylierungsreaktion beispielsweise Nitrobenzol, Schwefelkohlenstoff, Dichlormethan oder 1,2-Dichlorethan verwendet werden.

Diese Lösungsmittel werden in Mengen von 30 Massenprozent bis 2000 Massenprozent, bevorzugt von 100 Massenprozent bis 500 Massenprozent, bezogen auf die Menge des eingesetzten Benzoylhalogenids, verwendet. Es kann ökonomisch vorteilhaft sein, in Überschüssen von halogeniertem Benzol oder Benzoylhalogenid als Lösungsmittel zu arbeiten. Die Arbeitsweise ohne Zusatz eines inerten organischen Lösungsmittels ist bevorzugt.

Die Isolierung der Benzophenone nach der Acylierungsreaktion geschieht in im Prinzip bekannter Weise durch wäßrige Hydrolyse der Reaktionsmischungen und Extraktion oder Filtration der Produkte. Deren Reinigung kann durch Umkristallisation, Fraktionierung oder Chromatographie erfolgen. Bei Verwendung der metalldotierten Tonerdemineralien als Acylierungskatalysatoren kann man auch auf wäßrige Aufarbeitung verzichten. Man filtriert den Katalysator einfach ab und wäscht ihn unter Umständen mit Lösungsmittel. Die hierbei erhaltene Mutterlauge kann in der bereits beschriebenen Weise weiterverarbeitet werden, wobei Abdestillieren des Lösungsmittels und Ausfällen des Produktes mit Wasser auch hier eine günstige Alternative darstellt.

Bei der erfindungsgemäßen Acylierungsreaktion erhält man die Benzophenone in der Regel in Ausbeuten von etwa 70 % bis etwa 95 %, abhängig vom Substitutionsmuster. Die erhaltenen Benzophenone können direkt oder erst nach anschließender Halex-Reaktion in die Umsetzung zu wertvollen halogenierten Benzoesäuren und Phenolen eingesetzt werden.

Bei der sich ggfs. anschließenden Fluorierung der nach der Acylierungsreaktion angefallenen halogenierten Benzophenone durch Reaktion mit den weiter oben genannten Alkalimetallfluoriden (Halex-Reaktion) kann man in dipolar aprotischen Lösungsmitteln wie Sulfolan (Tetramethylensulfon), Tetramethylensulfoxid, N,N-Diethylacetamid, N,N-Dimethylacetamid, N,N-Dimethylformamid, N-Methylpyrrolidon, Dimethylsulfoxid, Dimethylsulfon, Diphenylsulfoxid, Diphenylsulfon, Tetramethylharnstoff, Tetra-n-butylharnstoff, 1,3-Dimethylimidazolidin-2-on oder Mischungen daraus oder auch ohne jedes Lösungsmittel arbeiten. Bevorzugt ist die Verwendung von N,N-Dimethylacetamid, Sulfolan (Tetramethylensulfon), 1,3-Dimethylimidazolidin-2-on oder Diphenylsulfon sowie die Arbeitsweise ohne Lösungsmittel. Besonders bevorzugt ist es, in Diphenylsulfon oder ohne Lösungsmittel zu arbeiten oder sonstige Lösungsmittel zu verwenden, die keine aliphatisch gebundenen Wasserstoffatome enthalten. Wie schon erwähnt, kann es in einzelnen Fällen zweckmäßig sein, in Mischungen der angegebenen Lösungsmittel zu arbeiten.

Arbeitet man ohne dipolar aprotisches Lösungsmittel in der Schmelze des erfindungsgemäßen Einsatzmaterials, so ist es in diesen Fällen auch zweckmäßig, mit Fluoridsalzen im Unterschuß zu arbeiten, um Rührbarkeit zu gewährleisten. Die Salzmengen können in diesem Fall somit 50 Molprozent bis 100 Molprozent Alkalimetallfluorid pro auszutauschendem Chloratom betragen. Mischungen aus Kalium- und Cäsiumfluorid und reines Kaliumfluorid sind bevorzugt. Das erfindungsgemäße Verfahren toleriert die Verwendung von sprühgetrocknetem Fluoridsalz, die aber zum Erhalt guter Ergebnisse nicht erforderlich ist.

Als Phasentransferkatalysatoren kommen quartäre Ammonium- oder Phosphoniumverbindungen, wie Tetraalkyl($C_1$-$C_{18}$)-ammoniumchloride, -bromide oder -fluoride, Tetraalkyl($C_1$-$C_{18}$)-phosphoniumchloride oder bromide, Tetraphenylphosphoniumchlorid oder -bromid, ((Phenyl)$_m$(alkyl($C_1$-$C_{18}$))$_n$)-phosphoniumchloride oder -bromide, wobei $m$ = 1 bis 3, $n$ = 3 bis 1 und $m + n = 4$ ist, oder Mischungen derselben in Frage. Bevorzugt sind hierbei Phosphoniumsalze, besonders bevorzugt Tetraphenylphosphoniumsalze oder sonstige Verbindungen, die keine aliphatisch gebundenen Wasserstoffatome enthalten. Diese Stoffe werden in Mengen von 0,01 bis 50 Molprozent, bezogen auf eingesetztes Benzophenon der allgemeinen Formel 1 eingesetzt, bevorzugt zwischen 0,5 bis 10 Molprozent, besonders bevorzugt zwischen 1 und 5 Molprozent.

Ferner können als Phasentransferkatalysatoren Oligo- oder Polyethylenglykoldimethylether verwendet werden. Die Zahl der Glykoleinheiten in diesen Verbindungen kann von $n = 4$ (Tetraethylenglykoldimethylether) bis $n = 150$ betragen. Bevorzugt werden jedoch Ether eingesetzt, deren Polymerisationsgrad zwischen $n = 4$ und $n = 25$ beträgt. Die optimale Einsatzmenge dieser Glykolether liegt zwischen 0,5 Massenprozent und 200 Massenprozent, bezogen auf die Massen des eingesetzten Reaktionssalzes, bevorzugt zwischen 5 und 100 Massenprozent, besonders bevorzugt zwischen 10 und 50 Massenprozent. Der besondere Vorteil bei der Verwendung dieser Verbindungen liegt darin, daß in der Regel, der Einsatzmenge entsprechend, weniger Lösungsmittel verwendet werden kann, weil die Glykolether bei der Reaktionstemperatur stets flüssig sind. In einigen Fällen erzielt man besonders günstig Ergebnisse, wenn man eine Mischung von vorstehend spezifizierten Phasentransferkatalysatoren verwendet, insbesondere Mischungen von Ammonium- oder Phosphoniumsalzen mit Polyethylenglykoldimethylethern.

Das nach der Chlor-Fluor-Austauschstufe erhaltene Produktgemisch wird in der Regel durch Filtration des Reaktionssalzes, besonders bei Durchführung im technischem Maßstab, und anschließende direkte Fraktionierung des Produktes aus dem Filtrat aufgearbeitet. Ebenfalls möglich ist, das Rohgemisch mit Wasser zu versetzen und die leichtere Oberphase, die das Produkt enthält, abzutrennen. Durch Extraktion des Wassers kann eine vollständige Abtrennung des Produktes aus der Mutterlauge erhalten werden. Danach kann durch chromatographische oder destillative Trennung eine Reinigung erfolgen. Besonders einfach ist es im vorliegenden Fall nach Filtration des Reaktionssalzes das

Lösungsmittel abzudestillieren und das Produkt durch Zugabe von Wasser auszufällen und zu filtrieren.

Die Ausbeuten der Chlor-Fluor-Austausch-Stufe betragen etwa 65 bis etwa 85 %, je nach Wahl des Lösungsmittels, des Katalysators, der Reaktionstemperatur und der Konzentration im Lösungsmittel.

Das erfindungsgemäße Verfahren im Umfang des Chlor-Fluor-Austausches kann in der beschriebenen Weise ebenso auf symmetrische Benzophenone angewendet werden. So kann man beispielsweise aus 4,4'-Dichlorbenzophenon das 4,4'-Difluorbenzophenon und aus 2,2',4,4'-Tetrachlorbenzophenon das 2,2'-Dichlor-4,4'-difluorbenzophenon herstellen. Diese Verbindungen lassen sich somit gegenüber bekannten Verfahren in reaktionstechnisch und ökonomisch verbesserter Weise herstellen.

Das Verfahren kann in beiden Reaktionsstufen bei Atomsphärendruck, Unter- oder Überdruck durchgeführt werden. Die Verfahrensweise bei Atmosphärendruck ist jedoch jeweils bevorzugt.

Abgesehen davon, daß es sich bei den erfindungsgemäßen Verbindungen um neue, asymmetrische, halogenierte Benzophenone, die wertvolle Zwischenprodukte zur Herstellung von Pharmazeutika, Pflanzenschutzmitteln und Flüssigkristallen darstellen, handelt, war es nach dem Stand der Technik nicht zu erwarten, daß die neuen Benzophenone auf befriedigende Weise hergestellt werden können. So ist zum Beispiel aus der Literatur (Methoden der organischen Chemie (Houben-Weyl-Müller) Band 7/2a, 43 (1973), Thieme-Verlag, Stuttgart) bekannt, daß Acylierungen an Polyhalogenbenzolen nicht oder nur sehr schwierig durchzuführen sind.

Besondere Schwierigkeiten machte bis jetzt die Herstellung der fluorierten Benzophenone, die zum Teil aufgrund ihres Substitutionsmusters auf literaturbekannten Wegen nicht zugänglich waren oder zu deren Synthese äußerst kostspielige, bereits fluorierte aromatische Vorprodukte notwendig waren.

Die nachstehende Beispiele dienen zur Erläuterung des Verfahrens, ohne es darauf zu beschränken.

Allgemeine Arbeitsvorschrift zur Herstellung erfindungsgemäßer Benzophenone durch Friedel-Crafts-Acylierung:

Man setzt 1 Mol Säurechlorid in Anwesenheit von 1,5 bis 3 Equivalenten Acylierungskatalysator mit einem Halogenaromaten um, der im allgemeinen als Lösungsmittel (3-5 Mol) verwendet wird (ersatzweise verwendet man 1 Mol Halogenaromat und die entsprechende Menge Lösungsmittel). Das (substituierte) Benzoylchlorid wird hierbei bei Reaktionstemperatur zugetropft. Die Reaktionstemperatur beträgt bei Umsetzung von Monohalogenaromaten zwischen 0 °C und 50 °C, bei Dihalogenaromaten zwischen 40 °C und 80 °C, bei Trihalogenaromaten zwischen 80 °C und 130 °C und bei Tetrahalogenaromaten zwischen 120 °C und 180 °C. Die Umsetzung wird GC-analytisch überwacht und in der Regel nach 3 bis 20 h abgebrochen, die Reaktionsmischung mit Eis hydrolysiert. Durch Zugabe eines Extraktionsmittels kann die anschließende Phasentrennung verbessert werden. Die organische Phase wird getrocknet, und kann durch Einengen oder Verdünnen mit anderen Lösungsmitteln (Methanol) zum Kristallisieren gebracht werden. Flüssige Produkte werden durch Destillation des beim vollständigen Entfernen des Lösungsmittels verbleibenden Rückstandes erhalten.

Auf diese Weise können u.a. hergestellt werden:

2,4-Dichlorbenzophenon (beispielsweise aus 1,3-Dichlorbenzol und Benzoylchlorid, 82 % Ausbeute, Schmp. 51 °C);

Zum 2,4-Dichlorbenzophenon:

MS: m/z (%) = 51 (26), 77 (48), 105 (100), 145 (15), 147 (10), 173 (46), 175 (31), 177 (5), 215 (5), 250 (55), 252 (40)

Es wurden 351 (4,5 Mol) Benzol und 360 g (2,7 Mol) Aluminiumchlorid bei 40 °C vorgelegt und 187,5 g (0,895 Mol) 2,4-Dichlorbenzoylchlorid in 50 min zugetropft. Nach beendeter Gasentwicklung zersetzte man mit 2 l Eiswasser und destillierte das Benzol azeotrop ab. Das Produkt wurde in Wasser granuliert und mit 1000 ml Methanol verrührt. Die Lösung wurde anschließend etwas eingeengt. Bei 50 °C setzte man Petrolether zu, ließ kristallisieren, saugte ab und wusch mit Petrolether. Man erhielt 183,4 g (0,734 Mol, 82 %) 2,4-Dichlorbenzophenon vom Schmelzpunkt 51 °C (Reingehalt > 99 %).

2,2',4,4'-Tetrachlorbenzophenon (aus 2,4-Dichlorbenzoylchlorid und 1,3-Dichlorbenzol, 93 % Ausbeute, Schmp. 79 °C).

Zum 2,2',4,4'-Tetrachlorbenzophenon:

661,5 g (4,5 Mol) 1,3-Dichlorbenzol und 360 g (2,7 Mol) Aluminiumchlorid wurden bei 40 - 50°C vorgelegt und 187,5 g (0,895 Mol) 2,4-Dichlorbenzoylchlorid zugetropft. Nach beendeter Gasentwicklung wurde mit Eis/Salzsäure hydrolysiert und danach azeotrop abdestilliert (Dichlorbenzol/Wasser). Das entstandene Produkt wurde in der Wasserphase granuliert und mit 600 ml 50 %igem Methanol gewaschen. Man erhielt 267,6 g (0,832 Mol, 93 %) 98%iges Produkt (GC).

Schmp. 79 °C

MS: m/z (%) = 74 (18), 109 (25), 145 (28), 147 (19), 173 (100), 175 (74), 220 (3), 248 (1), 283 (2), 318 (40), 320 (52), 322 (29)

Einzelbeispiele 1 - 22 zur Herstellung der erfindungsgemäßen Benzophenone durch Friedel-Crafts-Acylierung

Beispiel 1

Zu 226,9 g (1,25 Mol) 1,2,3-Trichlorbenzol und 166,6 g (1,25 Mol) Aluminiumchlorid wurden bei 130 °C 79,3 g (0,5 Mol) 4-Fluorbenzoylchlorid so zugetropft, daß die Temperatur bei 130 °C gehalten werden konnte. Man heizte solange bei dieser Temperatur nach, bis die Gasentwicklung beendet war. Die Mischung wurde danach 8 h bei dieser Temperatur gehalten und anschließend auf 1000 g Eis gegeben und mit 300 g Toluol versetzt. Nach Phasentrennung wurde die organische Phase zweimal mit je 200 ml 5 %iger Natriumhydrogencarbonatlösung gewaschen. Das Lösungsmittel wurde azeotrop abdestilliert und überschüssiges 1,2,3-Trichlorbenzol aus dem Rückstand durch Wasserdampfdestillation entfernt. Die verbleibende Substanz wurde durch Ausrühren bei 80 °C granuliert. Es wurden 158,6 g (feucht) bzw. 148,0 g (0,49 Mol, 97,5 %) 2,3,4-Trichlor-4'-fluorbenzophenon (trocken) erhalten.

Bei Verwendung von 333,4 g (1,25 Mol) Aluminium(III)-bromid oder 74,6 g (1,1 Mol) Bortrifluorid und/oder 300 ml $CS_2$ (40°C/24 h) oder 300 ml 1,2-Dichlorethan (85°C/16 h) als Lösungsmittel erhält man ähnliche Ausbeuten und Selektivitäten.

Zum erhaltenen 2,3,4-Trichlor-4'-fluorbenzophenon:

Schmp. 74 - 76 °C

$^1$H-NMR ($CDCl_3$, TMS):

$\delta$ = 7.16 (cm, 2H, Ar-$\underline{H}^{3',5'}$)
7.21 (d, 1H, Ar-$\underline{H}^5$)
7.51 (d, 1H, Ar-$\underline{H}^6$)
7.82 (cm, 1H, Ar-$\underline{H}^{2',6'}$)

$^{19}$F-NMR ($CDCl_3$, $CFCl_3$):

$\delta$ = -103,36 (dd, 1F, J = 13,5 Hz, J = 5,5 Hz, J = 8,4 Hz, J = 2,9 Hz, Ar-$\underline{F}^{4'}$)

MS: m/z (%) = 75 (17), 95 (36), 109 (5), 123 (100), 145 (4), 179 (7), 181 (7), 211 (5), 267 (2), 302 (36, $M^+$), 304 (37), 306 (13)

Beispiel 2

55,5 g (0,35 Mol) 2-Fluorbenzoylchlorid, 175,8 g (1,03 Mol) 1,2,4-Trichlorbenzol und 136,6 g (1 Mol) Zink(II)-chlorid wurden 10 h auf 140 °C erhitzt. Danach wurde mit 500 g Eis zersetzt, 100 ml Dichlormethan zugegeben und getrennt. Die organische Phase wurde mit 10 g 10%iger Natronlauge gewaschen und das Produkt durch Zugabe von Methanol ausgefällt (48,8 g Rohprodukt, 0,16 Mol, 46 %). Duch Umlösen aus 210 g Methanol unter Zusatz von 4 g Aktivkohle wurden 25,0 g (82 mMol, 24 %) 2,4,5-Trichlor-2'-fluorbenzophenon als farbloser Feststoff erhalten.

Bei Verwendung von 105,1 g (0,7 Mol) Trifluormethansulfonsäure und 400 ml 1,2-Dichlorethan (85 °C) als Lösungsmittel erhält man ähnliche Ausbeuten und Selektivitäten (8 h Reaktionszeit). Schmp. 84 °C.

Zum erhaltenen 2,4,5-Trichlor-2'-fluorbenzophenon:

$^1$H-NMR (DMSO-$d_6$, TMS):
$\delta$ = 7.36 (s, 1H, $J_F$ = 11.5 Hz, Ar$\underline{H}^{3'}$)
7.39 (ddd, 1H, $J_F$ = 7.6 Hz, Ar-$\underline{H}^{6'}$)
7.7-7.8 (m, 2H, $J_F$ = 5.2 Hz, Ar-$\underline{H}^{4',5'}$)
7.92 (s, 1H, Ar-$\underline{H}^3$)
8.01 (s, 1H, Ar-$\underline{H}^6$)
$^{19}$F-NMR (DMSO-$d_6$, $CFCl_3$):
$\delta$ = -111.0 (dddd, 1F, J = 5.2 Hz, J = 7.6 Hz, J = 11.5 Hz, Ar-$\underline{F}^2$)
MS: m/z (%) = 75 (17), 95 (27), 109 (5), 123 (100), 143 (7), 179 (10), 181 10), 207 (30), 209 (30), 211 (10), 267 (2),

302 (41, $M^+$), 304 (42), 306 (19)

Beispiel 3

Zu 7,8 g (0,03 Mol) 2,3,4,5-Tetrachlorbenzoesäure in 48 g (0,5 Mol) Fluorbenzol wurden bei 20-25 °C 7,1 g (0,06 Mol) Thionylchlorid getropft und die Temperatur allmählich auf 80 °C erhöht. Nach 6 h zieht man überschüssiges Thionylchlorid und einiges Fluorbenzol bei 200 mbar ab und gibt 10 g (0,075 Mol) Aluminiumchlorid bei 20 °C in Portionen unter Kühlung zu. Anschließend heizt man 4 h auf 40-50 °C und hydrolysiert danach durch Aufgießen auf 250 g Eis. Die wäßrige Phase wurde mit Methyl-tert.butylether extrahiert, über $MgSO_4$ getrocknet und das Lösungsmittel und überschüssiges Fluorbenzol am Rotationsverdampfer entfernt. Es wurden 8,4 g Rohprodukt (24,9 mMol, 83 %) erhalten, das aus Ethanol/Wasser umkristallisiert wurde.

Bei Verwendung von 21,5 g (0,125 Mol) p-Toluolsulfonsäure und 350 ml Dichlormethan (25 °C) als Lösungsmittel erhält man ähnliche Ausbeuten und Selektivitäten.

Zum erhaltenen 2,3,4,5-Tetrachlor-4'-fluorbenzophenon:

Schmp. 104,5 - 106 °C
$^1$H-NMR ($CDCl_3$, TMS):
$\delta$ = 7.17 (cm, 2H, Ar-$\underline{H}^{3',5'}$)
7.40 (s, 1H, Ar-$\underline{H}^{6}$)
7.83 (cm, 2H, Ar-$\underline{H}^{2',6'}$)
$^{19}$F-NMR ($CDCl_3$, $CFCl_3$):
$\delta$ = -102.51 (dddd, 1F, J= 2,9 Hz, J = 5,3 Hz, J = 8,3 Hz, J = 13,6 Hz, Ar-$\underline{F}^{4'}$)
MS: m/z (%) = 75 (12), 95 (30), 123 (100), 180 (3), 215 (5), 241 (7), 243 (9), 303 (2), 336 (33, $M^+$), 338 (43), 340 (22) 342 (5)

Beispiel 4

In 33,3 g (0,2 Mol) 2,3,4-Trifluorchlorbenzol wurden 46,9 g (0,3 Mol) 2-Chlorbenzoesäure vorgelegt und bei 20 °C 41,7 g (0,35 Mol) Thionylchlorid zugetropft. Nach 3 h bei 80 °C wurde überschüssiges Thionylchlorid bei 200 mbar/60 °C abgezogen. Man gab 80 g (0,6 Mol) Aluminiumchlorid zu und steigerte die Temperatur langsam (1h) auf 150 °C. Nach 7 h bei Reaktionstemperatur arbeitete man analog zu Beispiel 6 auf und erhielt 53,4 g (0,175 Mol Rohprodukt) dunkelbraun gefärbten Rückstand, der die isomeren Produkte zu 82 GC-Flächen-% enthält.

Bei Verwendung von 179,4 g (0,6 Mol) Antimon(V)-chlorid oder 30,5 g (0,45 Mol) Bortrifluorid als Lösungsmittel erhält man ähnliche Ausbeuten und Selektivitäten.

Beispiel 5

Zu 15,1 (0,1 Mol) 1,2,3,4-Tetrafluorbenzol und 33,3 g (0,25 Mol) Aluminiumchlorid gab man 31,4 g (0,15 Mol) 3,4-Dichlorbenzoylchlorid zu und erhitzte unter Rühren zum Rückfluß (95 °C). Die Reaktionsmischung wurde nach 16 h mit 200 g Eis hydrolysiert, 2 h stehen gelassen und mit 100 ml Methyl-tert.butylether extrahiert. Die organische Phase wurde im Vakuum eingedampft und der Rückstand destilliert (1 mbar, 155 °C). Es ging ein farbloses, zähes Öl über, das beim Stehenlassen erstarrte (Schmp. 43,5 °C-48 °C). Man erhielt 28,6 g Rohprodukt (89 %, Gehalt (GC) 91 % Produkt, 3-4 % Isomerisierungsprodukte) und 21,2 g (65,5 mMol, 66 %) gereinigtes 3',4'-Dichlor-2,3,4,5-tetrafluorbenzophenon.

Zum erhaltenen 3',4'-Dichlor-2,3,4,5-tetrafluorbenzophenon:

$^1$H-NMR (DMSO-$d_6$, TMS):
$\delta$ = 7.70 (m, 1H, Ar-$\underline{H}^{6}$)
7.80 (dd, J = 8.4 Hz (H-5'), J = 2.0 Hz, Ar-$\underline{H}^{6'}$)
7.86 (dd, J = 8.4 Hz (H-6'), Ar-$\underline{H}^{5'}$)
8.00 (dd, J = 20 Hz, J = 0.4 Hz Ar-$\underline{H}^{2'}$)
$^{19}$F-NMR (DMSO-$d_6$, $CFCl_3$):
$\delta$ = -137.9 (m, 1F, Ar-$\underline{F}^{2,5}$)
-138.0 (m, 1F, Ar-$\underline{F}^{5,2}$)
-150.6 (m, 1F, Ar-$\underline{F}^{4}$)
-154.1 (m, 1F, Ar-$\underline{F}^{3}$)
MS: m/z (%) = 75 (20), 99 (31), 109 (22), 145 (35), 147 (27), 149 (40), 173 (100), 175 (72), 177 (83), 204 (3), 224 (8), 287 (12), 322 (95, $M^+$), 324 (70)

Beispiel 6

367 g (2,5 Mol) 1,2-Dichlorbenzol und 166,6 g (1,25 Mol) Aluminiumchlorid wurden vorgelegt und auf 110 °C erhitzt. Bei dieser Temperatur tropfte man innerhalb 1 h 79,3 g (0,5 Mol) 4-Fluorbenzoylchlorid zu und rührte 1 h bei 100 °C nach. Nach weiteren 2 h bei 120 °C wurde auf 600 g Eis gegossen, die Phasen getrennt, die organische Phase mit $Na_2CO_3$-Lösung gewaschen und danach ein Teil des Dichlorbenzols abdestilliert. Der Rückstand wurde bei 60 °C mit Petrolether verdünnt und das ausgefallene Produkt abgesaugt. Es wurden 91,7 g (0,34 Mol, 68 %) 3,4-Dichlor-4'-fluorbenzophenon als farbloser Festkörper vom Schmelzpunkt 92-93 °C erhalten. Der Isomerengehalt betrug etwa 0,4 % (NMR).

Zum erhaltenen 3,4-Dichlor-4'-fluorbenzophenon:

$^1$H-NMR ($CDCl_3$, TMS):

δ = 6.53 (tm, 2H, Ar-$\underline{H}^{3',5'}$)
6.92 (d, 1H, Ar-$\underline{H}^5$)
6.93 (d, 1H, Ar-$\underline{H}^6$)
7.16 (ddm(cm), 2H Ar-$\underline{H}^{2',6'}$)
7.20 (dd, 1H, Ar-$\underline{H}^2$)

$^{19}$F-NMR ($CDCl_3$, $CFCl_3$):

δ = -105.16 (dddd, 1F, Ar-$\underline{F}^{4'}$)

$^{13}$C-NMR [$CDCl_3$, TMS, ppm]: δ = 115.88 (Ar-$C^{3',5'}$, J = 21.7 Hz), 128.92, 130.61, 131.72, 132.62 (J = 9.30 Hz), 133.06 (J = 2.94 Hz), 133.22, 137.19, 137.24, 165.78 (Ar-$C^{4'}$, J = 255.86 Hz), 192.73 (Ar-$\underline{C}$O-Ar)

MS: m/z (%) = 50 (5), 75 (25), 95 (40), 109 (11), 123 (100), 145 (13), 147 (9), 173 (25), 175 (17), 204 (1), 233 (9), 268 (44, $M^+$), 270 (31)

Beispiel 7

308 g (1,87 Mol, 220 ml) 2,4-Dichlorfluorbenzol und 267 (2 Mol) Aluminiumchlorid wurden vorgelegt und auf 140 °C geheizt. Danach tropfte man 158 g (1 Mol) 4-Methylbenzoesäurechlorid innerhalb 1 h zu und rührte 7,5 h bei 140-150 °C nach. Die resultierende Mischung wurde mit Eis/Wasser-Dichlormethan (300/300/300 g) zersetzt und die organische Phase zweimal mit wäßriger $NaHCO_3$-Lösung gewaschen, mit $MgSO_4$ unter Zusatz von etwas Aktivkohle getrocknet und die Lösungsmittel bei 200 mbar und 170 °C Sumpftemperatur entfernt (111-115 °C Übergang). Durch Zugabe der doppelten Menge des Rückstandes an Methanol und Kühlen erhielt man in 3 Kristallfraktionen 141,0 g (50 %, 0,495 Mol) 2,4-Dichlor-5-fluor-4'-methylbenzophenon als leicht gelbliche Kristalle mit folgenden Daten:

Schmp. 70 - 72 °C
$^{19}$F-NMR (DMSO-$d_6$, $CFCl_3$):
δ = -116.4 (dd, 1F, J = 8.9 Hz, J = 6.5 Hz, Ar-$\underline{F}^5$)
$^1$H-NMR (DMSO-$d_6$, TMS):
δ = 2.40 (s, 3H, Ar-C$\underline{H}_3$(p))
7.37 (d, 2H, Ar-$\underline{H}^{6',2'}$)
7.67 (d, 2H, Ar-$\underline{H}^{3',5'}$)
7.71 (dd, 1H, J = 0.2 Hz, J = 8.9 Hz, Ar-$\underline{H}^6$)
7.97 (dd, 1H, J = 0.2 Hz, J = 6.5 Hz, Ar-$\underline{H}^3$)
MS: m/z (%) = 65 (17), 91 (33), 119 (100), 128 (5), 163 (9), 183 (6), 191 (11), 217 (1), 247 (2), 282 (40, $M^+$), 284 (29)
$^1$H-NMR [$CDCl_3$, TMS]:
δ = 2.43 (s, Ar-C$\underline{H}_3$)
7.17 (dd, 1H, J = 0.26 Hz, J = 8.27 Hz, Ar-$H^6$)
7.28 (m, 2H, Ar-$\underline{H}^{3',5'}$)
7.52 (dd, 2H, J = 0.31, J = 6.27 Ar-$\underline{H}^3$)
7.69 (m, 2H, Ar-$\underline{H}^{2',6'}$)

$^{19}$F-NMR [$CDCl_3$/ppm]:

δ = -116.96 (dd, 1F, J = 6.25 Hz, J = 8.15 Hz, Ar-F5)

$^{13}C$-NMR [$CDCl_3$, TMS, ppm]:

δ = 21.82 (Ar-$CH_3$), 117.09 ($^2J_{CF}$ = 23.79 Hz, Ar-$C^6$), 123.65 ($^2J_{CF}$ = 18.93 Hz, Ar-$C^4$), 126.92 ($^4J_{CF}$ = 4.25 Hz, Ar-$C^2$), 129.63 (Ar-$C^{2',6'}$), 130.27 (Ar-$C^{3',5'}$), 131.87 (Ar-$C^3$), 133.40 (Ar-$C^{1'}$), 138.80 ($^3J_{CF}$ = 5.39 Hz, Ar-$C^1$), 145.45 (Ar-$C^{4'}$), 156.80 ($^1J_{CF}$ = 252.41 Hz, Ar-$C^5$), 192.38 (Ar-CO-Ar)

Beispiel 8

In 17,8 g (0,15 Mol) Thionylchlorid und 100 g Chlorbenzol wurden 19,4 g (0,1 Mol) bei 20 °C 2,3,4,5-Tetrafluorbenzoesäure eingetragen. Die erhaltene beigefarbene Lösung wurde 3 h bei starkem Rückfluß auf 130 °C erhitzt und danach überschüssiges Thionylchlorid und etwas Chlorbenzol abdestilliert (40 g). Man gab bei 40 °C 35,5 g (0,25 Mol) Aluminiumchlorid zu. Nach 1 h wurde die orange-braune Reaktionsmischung auf 300 g Eis und 15 g Salzsäure (30 %) gegeben. Das sich abscheidende Öl wurde abgetrennt und die wäßrige Phase zweimal mit je 50 g Dichlormethan extrahiert, die organischen Phasen über $MgSO_4$ getrocknet. Nach Entfernen der Lösungsmittel verblieben 21,8 g beige-brauner Feststoff, der aus wäßrigem Ethanol umgelöst wurde. Man erhielt 18,5 g (64,2 mMol, 64 %) 2,3,4,5-Tetrafluor-4'-chlor-benzophenon als leicht gelbliches Pulver.

Zum erhaltenen 2,3,4,5-Tetrafluor-4'-chlorbenzophenon:

Schmp. 65,5-67 °C

MS: m/z (%) = 50 (10), 75 (28), 99 (20), 111 (40), 113 (14), 130 (6), 139 (100), 141 (32), 149 (21), 177 (29), 205 (2), 224 (3), 253 (6), 288 (84), 290 (29)

$^1H$-NMR ($CDCl_3$/ppm]:

δ = 7.22 (A) (dddd, 1H, $J_{AB}$ = 5.5 Hz, $J_{AC}$ = 9.33 Hz, $J_{AD}$ = 8.03 Hz, $J_{AE}$ = 2.65 Hz, Ar-$H^6$)

7.48 (m, 2H, Ar-$H^{3'}$)

7.74 (m, 2H, Ar-$H^{6'}$)

$^{19}F$-NMR [$CDCl_3$/ppm]:

δ = -136.05 (B) (dddt, 1F, $J_{AB}$ = 5.5 Hz, $J_{BC}$ = 13.35 Hz, $J_{BD}$ = 6.6 Hz, $J_{BE}$ = 21 Hz, Ar-$F^2$)

-137.12 (C) (dddd, 1F, $J_{CD}$ = 21 Hz, $J_{AC}$ = 9.33 Hz, $J_{BC}$ = 13.35 Hz, $J_{CE}$ = 3.55 Hz, Ar-$F^5$)

-149.50 (D) (dddd, 1F, $J_{AD}$ = 8.03 Hz, $J_{BD}$ = 6.6 Hz, $J_{CD}$ = 21 Hz, $J_{DE}$ = 19.3 Hz, Ar-$F^4$)

-153.36 (E) (dddd, 1F, $J_{AE}$ = 2.65 Hz, $J_{BE}$ = 21 Hz, $J_{DE}$ = 19.3 Hz, $J_{CE}$ = 3.55 Hz, Ar-$F^3$)

Beispiel 9

38 g (0,24 Mol) 4-Fluorbenzoylchlorid und 40 g (0,3 Mol) Aluminiumchlorid wurden in 300 ml $CS_2$ vorgelegt und bei 30 °C 25,9 g (0,24 Mol) Anisol zugetropft (45 min). Nach 2,5 h bei dieser Temperatur wurde mit Eiswasser/verd. Salzsäure/Dichlormethan hydrolysiert und die Lösemittel (Dichlormethan/$CS_2$) am Rotationsverdampfer entfernt. Der Rückstand wurde mit Methanol bei 15 °C verrührt, der ausgefallene Feststoff abgesaugt und mit Petrolether gewaschen (69,1 g Feuchtprodukt). Man erhielt 32,6 g (59 %, 0,142 Mol) Produkt vom Schmelzpunkt 91,5 - 92,5 °C.

MS: m/z (%) = 64 (8), 75 (11), 77 (15), 92 (13), 95 (24), 107 (10), 123 (21), 135 (100), 159 (4), 187 (4), 199 (9), 230 (65, $M^+$)

$^1H$-NMR [DMSO-$d_6$, TMS, ppm]:

δ = 3.85 (s, 3H, Ar-$OCH_3$), 7.09 (m, 2H, Ar-$H^{3',5'}$), 7.36 (m, 2H, Ar-$H^{3',5'}$), 7.74 (m, 2H, Ar-$H^{2',6'}$), 7.77 (m, 2H, Ar-$H^{2,6}$)

$^{19}F$-NMR [DMSO-$d_6$, $CDCl_3$, ppm]:

δ = -107.14 (dddd, 1F, J = 14.4 Hz, J = 8.9 Hz, J = 5.6 J = 3.3 Hz, Ar-$F^4$)

Allgemeine Arbeitsvorschrift zur Herstellung von Benzophenonen durch Chlor-Fluor-Austausch (Halex-)Reaktion: Die im folgenden beschriebenen Varianten der Umsetzung geben Verfahren wieder, die bei allen Verbindungen, die von Interesse sind, durchführbar sind und zu brauchbaren Produktausbeuten führen. Im einzelnen können die Verfahren für die speziellen Verbindungen hinsichtlich geringerer Salzüberschüsse, Katalysator- und Lösungsmittelmengen weiter optimiert werden.

A. Variante unter Verwendung von Lösungsmittel (Diphenylsulfon, Austausch eines Chloratoms)

Man legt 600 g Diphenylsulfon, 15 g Tetraphenylphosphoniumbromid, 43,5 g (0,75 Mol) Kaliumfluorid (im Falle besonders unreaktiver Verbindungen 46,4 g (0,75 Mol) Kaliumfluorid/Cäsiumfluorid (9:1 )) und 100 g Xylol vor und destilliert das Xylol bei Normaldruck vollständig ab. Sollte noch feuchtes Xylol zuletzt übergehen, setzt man solange frisches Xylol nach, bis keine Trübung mehr im Destillat entsteht. Man spült die Apparatur mit Inertgas (Argon), setzt 0,5 Mol des Chlor-benzophenons zu und heizt auf 220 °C. Im allgemeinen ist die Umsetzung nach 6-8 h beendet, es empfiehlt sich jedoch, diese gaschromatographisch zu überwachen und bei vollständigem Umsatz abzubrechen. Man setzt bei 130 °C unter Rühren 300 g 2-Chlortoluol zu und filtriert das Salz ab. Die Mutterlauge wird zunächst durch einfaches Abdestillieren vom 2-Chlortoluol befreit und der Rückstand, gegebenenfalls im Hochvakuum, fraktioniert.

Tauscht man zwei Chloratome gegen Fluoratome aus, so sind die Einsatzmengen von Lösungsmittel, Katalysator und Salz zu verdoppeln.

B. Variante ohne Verwendung eines Lösungsmittels (Austausch eines Chloratoms)

Man legt in 250 g Xylol 43,5 g (0,75 Mol) Kaliumfluorid (im Falle besonders unreaktiver Verbindungen 46,4 g (0,75 Mol) Kaliumfluorid/Cäsiumfluorid (9:1)) und 15 g Tetraphenylphosphoniumbromid vor und destilliert soviel Xylol ab, daß die Mischung gerade noch rührbar bleibt. Danach gibt man 0,5 Mol Chlorbenzophenon zu und heizt unter gleichzeitigem Abdestillieren des übrigen Xylols auf 220 °C. Die Umsetzung wird gaschromatographisch überwacht und im allgemeinen nach 6-8 h bei vollständigem Umsatz abgebrochen. Bei 130 °C gibt man 100 g 2-Chlortoluol zu und filtriert das Reaktionssalz ab, das nochmals mit dem Lösungsmittel gewaschen wird. Die Mutterlauge wird vom 2-Chlortoluol befreit und der Rückstand fraktioniert.

In wenigen Fällen ist es sinnvoll, 1 Mol Chlorbenzophenon bei sonst gleichbleibenden Einsatzmengen zu verwenden, um die Rührbarkeit zu gewährleisten. In diesem Falle gewinnt man überschüssig eingesetzes Chlorbenzophenon bei der Fraktionierung zurück. Tauscht man zwei Chloratome gegen Fluoratome aus, so sind die Einsatzmengen von Lösungsmitteln, Katalysator und Salz zu verdoppeln.

Auf die vorstehend beschriebenen Weisen können beispielsweise 3-Chlor-4-fluorbenzophenon (3,4-Dichlorbenzophenon), 4,4'-Difluorbenzophenon (aus 4,4'-Dichlorbenzophenon), 2-Chlor-4-fluorbenzophenon (aus 2,4-Dichlorbenzophenon), 2,4,4'-Trifluorbenzophenon aus 4,4'-Difluor-2-chlorbenzophenon, 2-Chlor-4-fluor-4'-methylbenzophenon (aus 2,4-Dichlor-4'-methylbenzophenon), 2,2'-Dichlor-4,4'-difluorbenzophenon (aus 2,2',4,4'-Tetrachlorbenzphenon), 2,3-Dichlor-4,4'-difluorbenzophenon (aus 2,3,4-Trichlor-4'-fluorbenzophenon) hergestellt werden.

Alternative Vorschrift zu 2,2'-Dichlor-4,4'-difluorbenzophenon (über Acylierung):

In 200 ml $CS_2$ wurden 61 g (0,5 Mol) 3-Chlorfluorbenzol und 200 g (1,5 Mol) Aluminiumchlorid vorgelegt. Dann tropfte man bei 35-41 °C 89,8 g (0,465 Mol) 2-Chlor-4-fluorbenzoylchlorid langsam zu. Als die Chlorwasserstoff-Entwicklung beendet war, wurde mit Eis, Dichlormethan und verdünnter Salzsäure zersetzt. Dann destillierte man aus der organischen Phase $CS_2$ und Dichlormethan mit Wasserdampf ab, wobei 72 g Rohprodukt erhalten wurden. Dieses wurde destilliert zu 54 g 81 %igem Produkt, das nach dreimaligem Umlösen aus wenig Methanol (40 - 25 ml) 97,8 %iges Material (GC) ergab.

Zum erhaltenen 2,2'-Dichlor-4,4'-difluorbenzophenon:

Schmp. 46,5 - 47,5 °C

$^1$H-NMR [$CDCl_3$/ppm]:

$\delta$ = 7.08 (C) (ddd, 2H, $J_{BC}$ = 2.44 Hz, $J_{CD}$ = 8.48 Hz, $J_{AC}$ = 8.68 Hz, Ar-$H^5$)
7.19 (B) (ddd, 2H $J_{BC}$ = 2.44 Hz, $J_{BD}$ = 7.70 Hz, $J_{AB}$ = 0.25 Hz, Ar-$H^3$)
7.56 (A) (ddd, 2H, $J_{AC}$ = 8.68 Hz, $J_{AD}$ = 6.0 Hz, $J_{AB}$ = 0.25 Hz, Ar-$H^6$)

$^{19}$F-NMR [$CDCl_3$/ppm]:

$\delta$ = -105.77 (D) (ddd, 2F, $J_{BD}$ = 7.70 Hz $J_{AD}$ = 6.0 Hz, $J_{CD}$ = 8.48 Hz, Ar-$F^4$)

MS: m/z (%) = 50 (5), 63 (3), 48 (4), 74 (7), 93 (12), 94 (14), 109 (10), 111 (5), 129 (34), 131 (11), 157 (100), 158 (34), 188 (5), 222 (0.7), 286 (31, $M^+$), 288 (21), 290 (4)

Einzelbeispiel 10 zur Herstellung der erfindungsgemäßen Benzophenone durch Chlor-Fluor-Austausch (Halex)-Reaktion:

Beispiel 10

Zu 350 g 1,3-Dimethylimidazolin-2-on gab man 3,1 g Tetraphenylphosphoniumbromid und 15,5 g (0,25 Mol) Kaliumfluorid/Cäsiumfluorid (9:1) und destillierte bei 155 °C im Vakuum 60 g des Lösungsmittels ab. Man heizte auf 215 °C und gab 33,6 g (0,125 Mol) 2,4-Dichlor-5-fluorbenzophenon (Isomerengehalt 11 %) zu. Die Mischung wurde unter

Argon 2,5 h gerührt und dann heiß vom Salz (18.9 g feucht, hellbraun) filtriert. Man destillierte sämtliches Lösungsmittel im Vakuum ab und fraktionierte den verbleibenden Rückstand über eine kurze Füllkörper-Kolonne. Man erhielt (17,7 g, 70,2 mMol, 56 %) 94 %iges 2-Chlor-4,5-difluorbenzophenon als gelbes Öl, das nach einiger Zeit erstarrte.

Führt man die Umsetzung bei 220 °C in Diphenylsulfon als Lösungsmittel anstatt in 1,3-Dimethylimidazolidin-2-on durch und verwendet lediglich 9,8 g Kaliumfluorid ohne Cäsiumfluorid-Zusatz, so beträgt die Reaktionszeit 6-8 h. Hierbei wird alles 2,4-Dichlor-5-fluorbenzophenon umgesetzt, sodaß 2,6-Dichlor-3-fluorbenzophenon unumgesetzt verbleibt und isoliert werden kann. Durch Erhöhung der Reaktionszeit (14 h) gelingt es, ca. 30 % des 2-Chlor-4,5-difluorbenzophenons zum 2,4,5-Trifluorbenzophenon umzusetzen. Die Trennung der Reaktionsgemische erfolgte durch Fraktionierung.

Setzt man Sulfolan als Lösungsmittel ein, verfährt jedoch sonst wie vorstehend beschrieben, so erhält man hauptsächlich dehalogenierte Produkte. Durch Verwendung entsprechender Überschüsse gelangt man so zu 3,4-Difluorbenzophenon.

Zum erhaltenen 2-Chlor-4,5-difluorbenzophenon:

Schmp. 65,6 °C

$^1$H-NMR (DMSO-$d_6$, TMS):

$\delta$ = 7.57 (t(m), 2H, Ar-$\underline{H}^{3'}$)
7.73 (t(m), 1H, Ar-$\underline{H}^{4'}$)
7.77 (d(m), 2H, Ar-$\underline{H}^{2'}$)
7.79 (dd(d), 1H, Ar-$\underline{H}^{6}$) (B) ($J_{AB}$ = 0.2 Hz, $J_{BC}$ = 8.2 Hz, $J_{BD}$ = 10.3 Hz)
7.90 (dd(d), 1H, Ar-$\underline{H}^{3}$) (A) ($J_{AB}$ = 0.2 Hz, $J_{AC}$ = 10.5 Hz, $J_{AD}$ = 7.0 Hz)

$^{19}$F-NMR (DMSO-$d_6$, $CFCl_3$):

$\delta$ = -132.6 (ddd, 1F, $J_{BC}$ = 8.2 Hz, $J_{AC}$ = 10.5 Hz, $J_{CD}$ = 20.3 Hz, Ar-$\underline{F}^4$)
-138.1 (dd, 1F, $J_{AD}$ = 7.0 Hz, $J_{BD}$ = 10.3 Hz, $J_{CD}$ = 20.3 Hz, Ar-$\underline{F}^5$) (D)

MS: m/z (%) = 51 (26), 77 (50), 97 (9), 105 (100), 112 (11), 147 (21), 149 (8), 175 (39), 177 (14), 188 (8), 217 (4), 252 (69, $M^+$), 254 (25)

2,4,5-Trifluorbenzophenon:

Sdp. 3 Torr/115 °C

$^1$H-NMR (DMSO-$d_6$, TMS):

$\delta$ = 7.55 (t(m), 2H, Ar-$\underline{H}^{3',5'}$) (E)
7.73 (t(m), 1H, Ar-$\underline{H}^{3}$) (D)
7.74 (d(m), 2H, Ar-$\underline{H}^{4'}$) (C) ($J_{CF}$ = 6.5 Hz, $J_{CG}$ = 8.9 Hz, $J_{CH}$ = 10.2 Hz)
7.77 (dd(d), 1H, Ar-$\underline{H}^{6}$) (B) ($J_{BF}$ = 9.3 Hz, $J_{BG}$ = 10.9 Hz, $J_{BH}$ = 6.4 Hz)
7.80 (dd(d), 2H, Ar-$\underline{H}^{2',6'}$) (A)

$^{19}$F-NMR (DMSO-$d_6$, $CFCl_3$):

$\delta$ = -113.1 (ddddd, 1F $J_{AF}$ = ca. 1 Hz $J_{CF}$ = 6.5 Hz, $J_{GF}$ = 6.6 Hz, $J_{BF}$ = 9.3 Hz, $J_{FH}$ = 15.8 Hz, Ar-$\underline{F}^2$) (F)
-128.8 (dddd, 1F, $J_{BG}$ = 10.9 Hz, $J_{CG}$ = 8.9 Hz, $J_{FG}$ = 6.6 Hz, $J_{HG}$ = 22.3 Hz, Ar-$\underline{F}^4$) (G)
-141.7 (dddd, 1F, $J_{BH}$ = 6.4 Hz, $J_{CH}$ = 10.2 Hz $J_{FH}$ = 15.8 Hz, $J_{BH}$ = 22.3 Hz, Ar-$\underline{F}^5$) (H)

MS: m/z (%) = 50 (11), 51 (29), 77 (58), 81 (25), 105 (100), 131 (30), 159 (55), 188 (3), 206 (3), 216 (5), 236 (93, $M^+$), 237 (14)

Zum 3,4-Difluorbenzophenon:

MS: m/z (%) = 49 (9), 51 (22), 63 (19), 77 (48), 95 (5), 105 (97), 113 (45), 141 (57), 188 (8), 198 (4), 218 ($M^+$, 100), 219 (16)

## Patentansprüche

1. Asymmetrische halogenierte Benzophenone der allgemeinen Formel

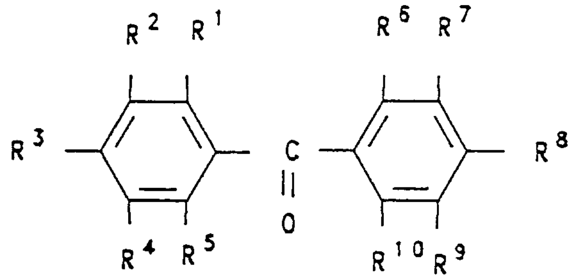

(1)

worin $R^1$, $R^3$ für Chlor, $R^4$ für Fluor oder $R^1$ für Chlor oder Fluor, $R^3$, $R^4$ für Fluor oder $R^1$ für Chlor, $R^3$ für Fluor, $R^4$ für Wasserstoff und $R^2$, $R^5$, $R^6$, $R^7$, $R^8$, $R^9$ und $R^{10}$ für Wasserstoff oder $R^1$ für Chlor, $R^3$, $R^4$ und $R^8$ für Fluor und $R^2$, $R^5$, $R^6$, $R^7$, $R^9$ und $R^{10}$ für Wasserstoff oder $R^1$, $R^2$, $R^3$ für Chlor, $R^4$ für Chlor oder Wasserstoff, $R^8$ für Fluor und $R^5$, $R^6$, $R^7$, $R^9$ und $R^{10}$ für Wasserstoff oder $R^1$, $R^3$, $R^4$ für Chlor, $R^6$ für Fluor und $R^2$, $R^5$, $R^7$, $R^8$, $R^9$ und $R^{10}$ für Wasserstoff oder $R^1$, $R^6$ für Chlor, $R^2$, $R^3$, $R^4$ für Fluor, $R^5$, $R^7$, $R^8$, $R^9$ und $R^{10}$ für Wasserstoff oder $R^1$, $R^2$, $R^3$, $R^4$ für Fluor, $R^7$ für Chlor oder Wasserstoff, $R^8$ für Chlor, und $R^5$, $R^6$, $R^9$ und $R^{10}$ für Wasserstoff oder $R^2$, $R^3$ für Chlor, $R^8$ für Fluor und $R^1$, $R^4$, $R^5$, $R^6$, $R^7$, $R^9$ und $R^{10}$ für Wasserstoff oder $R^1$, $R^3$ für Chlor, $R^4$ für Fluor, $R^8$ für Methyl und $R^2$, $R^5$, $R^6$, $R^7$, $R^9$ und $R^{10}$ für Wasserstoff oder $R^3$ für Fluor, $R^8$ für Methoxy, $R^1$, $R^2$, $R^4$, $R^5$, $R^6$, $R^7$, $R^9$ und $R^{10}$ für Wasserstoff oder $R^2$, $R^3$ für Fluor, $R^1$ und $R^4$ bis $R^{10}$ für Wasserstoff stehen.

2. Verbindung nach Anspruch 1 entsprechend der Formel

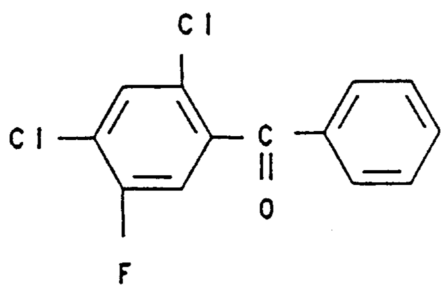

3. Verbindung nach Anspruch 1 entsprechend der Formel

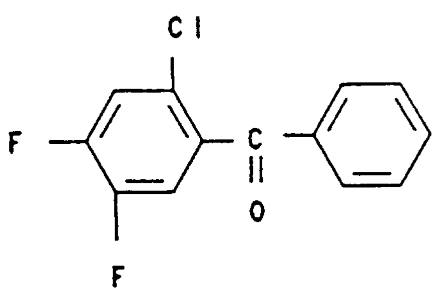

4. Verbindung nach Anspruch 1 entsprechend der Formel

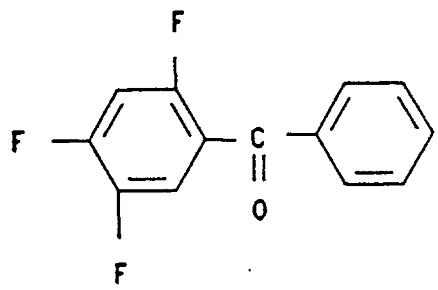

5. Verbindung nach Anspruch 1 entsprechend der Formel

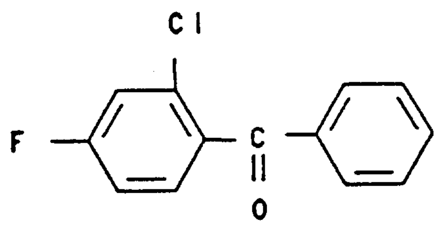

6. Verbindung nach Anspruch 1 entsprechend der Formel

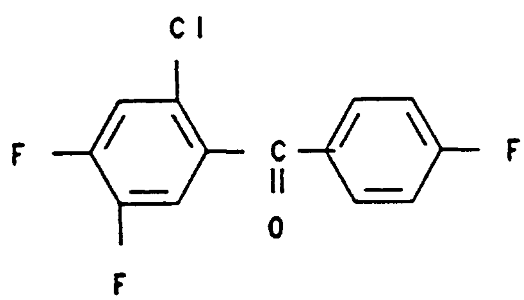

7. Verfahren zur Herstellung von asymmetrischen halogenierten Benzophenonen der allgemeinen Formel

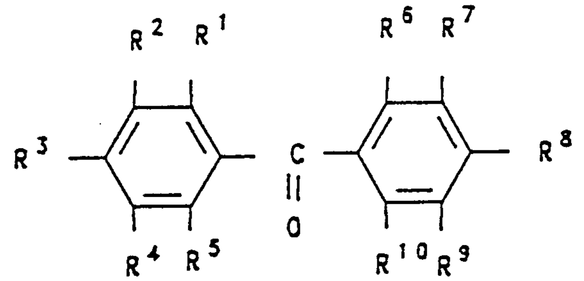

(1)

worin $R^1$, $R^3$ für Chlor, $R^4$ für Fluor oder $R^1$ für Chlor oder Fluor, $R^3$, $R^4$ für Fluor oder $R^1$ für Chlor, $R^3$ für Fluor, $R^4$ für Wasserstoff und $R^2$, $R^5$, $R^6$, $R^7$, $R^8$, $R^9$ und $R^{10}$ für Wasserstoff oder $R^1$ für Chlor, $R^3$, $R^4$ und $R^8$ für Fluor und $R^2$, $R^5$, $R^6$, $R^7$, $R^9$ und $R^{10}$ für Wasserstoff oder $R^1$, $R^2$, $R^3$ für Chlor, $R^4$ für Chlor oder Wasserstoff, $R^8$ für Fluor

und $R^5$, $R^6$, $R^7$, $R^9$ und $R^{10}$ für Wasserstoff oder $R^1$, $R^3$, $R^4$ für Chlor, $R^6$ für Fluor und $R^2$, $R^5$, $R^7$, $R^8$, $R^9$ und $R^{10}$ für Wasserstoff oder $R^1$, $R^6$ für Chlor, $R^2$, $R^3$, $R^4$ für Fluor, $R^5$, $R^7$, $R^8$, $R^9$ und $R^{10}$ für Wasserstoff oder $R^1$, $R^2$, $R^3$, $R^4$ für Fluor, $R^7$ für Chlor oder Wasserstoff, $R^8$ für Chlor, und $R^5$, $R^6$, $R^9$ und $R^{10}$ für Wasserstoff oder $R^2$, $R^3$ für Chlor, $R^8$ für Fluor und $R^1$, $R^4$, $R^5$, $R^6$, $R^7$, $R^9$ und $R^{10}$ für Wasserstoff oder $R^1$, $R^3$ für Chlor, $R^4$ für Fluor, $R^8$ für Methyl und $R^2$, $R^5$, $R^6$, $R^7$, $R^9$ und $R^{10}$ für Wasserstoff oder $R^3$ für Fluor, $R^8$ für Methoxy, $R^1$, $R^2$, $R^4$, $R^5$, $R^6$, $R^7$, $R^9$ und $R^{10}$ für Wasserstoff oder $R^2$, $R^3$ für Fluor, $R^1$ und $R^4$ bis $R^{10}$ für Wasserstoff stehen, dadurch gekennzeichnet, daß man 1 Mol eines halogenierten Benzols der allgemeinen Formel (2)

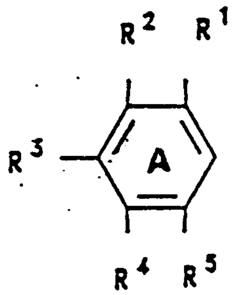

(2)

in welcher $R^1$, $R^2$, $R^3$, $R^4$, $R^5$ die vorstehend genannten Bedeutungen besitzen mit 1 bis 5 Mol eines Benzoylhalogenids der allgemeinen Formel (3)

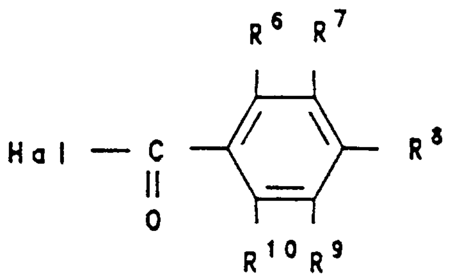

(3)

in welcher Hal ein Fluor-, Chlor- oder Bromatom und $R^6$, $R^7$, $R^8$, $R^9$ und $R^{10}$ die vorstehend genannten Bedeutungen haben, in Gegenwart eines Acylierungskatalysators bei Temperaturen von 0 °C bis 230 °C in Abwesenheit oder in Gegenwart eines gegenüber den Reaktionsteilnehmern inerten organischen Lösungsmittels acyliert und ggfs. die so erhaltenen Benzophenone der genannten Formel (1), sofern mindestens einer der Substituenten $R^1$ - $R^{10}$ ein Chloratom darstellt, ggfs. nach ihrer Zwischenisolierung mit 1 bis 2,5 Mol eines Kalium-, Rubidium- oder Cäsiumfluorids oder Mischungen davon pro auszutauschendem Chloratom bei Temperaturen von 120 bis 280 °C in Abwesenheit oder in Gegenwart eines Phasentransferkatalysators und in Abwesenheit oder in Gegenwart eines dipolar aprotischen Lösemittels umsetzt.

**8.** Verfahren nach Anspruch 7, dadurch gekennzeichnet, daß man die Acylierung bei Temperaturen von 70 °C bis 150 °C durchführt.

**9.** Verfahren nach mindestens einem der Ansprüche 7 und 8, dadurch gekennzeichnet, daß man Aluminiumchlorid, Aluminiumbromid, Antimon(V)-chlorid, Eisen(III)-chlorid, Eisen(II)-chlorid, Titan(IV)-chlorid, Bortrifluorid, Zinn(IV)-chlorid, Wismut(III)-chlorid, Zinkchlorid, Quecksilber(II)-chlorid, Fluorwasserstoffsäure, Schwefelsäure, Polyphosphorsäure, p-Toluolsulfonsäure oder Fluoralkansulfonsäuren als Acylierungskatalysatoren verwendet.

**10.** Verfahren nach mindestens einem der Ansprüche 7 bis 9, dadurch gekennzeichnet, daß man Aluminiumtrichlorid als Acylierungskatalysator verwendet.

**11.** Verfahren nach mindestens einem der Ansprüche 7 bis 10, dadurch gekennzeichnet, daß man in Nitrobenzol, Schwefelkohlenstoff, Dichlormethan oder 1,2-Dichlorethan als inertem organischen Lösungsmittel acyliert.

**12.** Verfahren nach mindestens einem der Ansprüche 7 bis 11, dadurch gekennzeichnet, daß man die Acylierungskatalysatoren in Mengen von 100 bis 500 Molprozent, bezogen auf eingesetztes Benzoylhalogenid, anwendet.

**13.** Verfahren nach mindestens einem der Ansprüche 7 bis 12, dadurch gekennzeichnet, daß man die Acylierungskatalysatoren in Mengen von 200 bis 300 Molprozent, bezogen auf eingesetztes Benzoylhalogenid, anwendet.

**14.** Verfahren nach mindestens einem der Ansprüche 7, 8 und 11, dadurch gekennzeichnet, daß man metalldotierte Tonerdemineralien als Acylierungskatalysatoren verwendet.

**15.** Verfahren nach mindestens einem der Ansprüche 7, 8, 11 und 14, dadurch gekennzeichnet, daß man die metalldotierten Tonerdemineralien in Mengen von 2 bis 100 Massenprozent, bezogen auf eingesetztes Benzoylhalogenid, verwendet.

**16.** Verfahren nach mindestens einem der Ansprüche 7, 8, 11, 14 und 15, dadurch gekennzeichnet, daß man die metalldotierten Tonerdemineralien in Mengen von 10 bis 30 Massenprozent, bezogen auf eingesetztes Benzoylhalogenid, verwendet.

**17.** Verfahren nach mindestens einem der Ansprüche 7, 8, 11, 14, 15 und 16, dadurch gekennzeichnet, daß man die Acylierungsreaktion in Gegenwart von metalldotierten Tonerdemineralien bei Temperaturen von 140 °C bis 220 °C durchführt.

**18.** Verfahren nach mindestens einem der Ansprüche 7 bis 17, dadurch gekennzeichnet, daß man als Phasentransferkatalysator quartäre Ammonium- oder Phosphoniumverbindungen einsetzt.

**19.** Verfahren nach mindestens einem der Ansprüche 7 bis 18, dadurch gekennzeichnet, daß man als Phasentransferkatalysator Tetraalkyl($C_1$-$C_{18}$)-ammoniumchloride, -bromide oder -fluoride, Tetraalkyl-($C_1$-$C_{18}$)-phosphoniumchloride, oder -bromide, Tetraphenylphosphoniumshlorid oder - bromid, ((Phenyl)$_m$(alkyl($C_1$-$C_{18}$))$_n$)-phosphoniumchloride oder -bromide, wobei m = 1 bis 3, n = 3 bis 1 und m + n = 4 sind, verwendet.

**20.** Verfahren nach mindestens einem der Ansprüche 7 bis 19, dadurch gekennzeichnet, daß man den Phasentransferkatalysator in einer Menge von 0,01 bis 50 Molprozent, bezogen auf das halogenierte Benzophenon, einsetzt.

**21.** Verfahren nach mindestens einem der Ansprüche 7 bis 20, dadurch gekennzeichnet, daß man den Phasentransferkatalysator in einer Menge von 0,5 bis 10 Molprozent, bezogen auf das halogenierte Benzophenon, einsetzt.

**22.** Verfahren nach mindestens einem der Ansprüche 7 bis 21, dadurch gekennzeichnet, daß man den Phasentransferkatalysator in einer Menge von 1 bis 5 Molprozent, bezogen auf das halogenierte Benzophenon, einsetzt.

**23.** Verfahren nach mindestens einem der Ansprüche 7 bis 17, dadurch gekennzeichnet, daß man als Phasentransferkatalysator Oligo- oder Polyethylenglykoldimethylether mit 4 bis 150 Glykoleinheiten verwendet.

**24.** Verfahren nach mindestens einem der Ansprüche 7 bis 17 und 23, dadurch gekennzeichnet, daß man als Phasentransferkatalysator Oligo- oder Polyethylenglykoldimethylether mit 3 bis 25 Glykoleinheiten verwendet.

**25.** Verfahren nach mindestens einem der Ansprüche 7 bis 17, 23 und 24, dadurch gekennzeichnet, daß man als Phasentransferkatalysator Oligo- oder Polyethylenglykoldimethylether in Mengen von 0,5 Massenprozent bis 200 Massenprozent` bezogen auf die Masse des eingesetzten Reaktionssalzes, verwendet.

**26.** Verfahren nach mindestens einem der Ansprüche 7 bis 17 und 23 bis 25, dadurch gekennzeichnet, daß man die Oligo- oder Polyethylenglykolether in Mengen von 5 bis 100 Massenprozent, bezogen auf die Masse des eingesetzten Fluoridsalzes verwendet.

**27.** Verfahren nach mindestens einem der Ansprüche 7 bis 17 und 23 bis 26, dadurch gekennzeichnet, daß man die Oligo- oder Polyethylenglykolether in Mengen von 10 bis 50 Massenprozent, bezogen auf die Masse des eingesetzten Fluoridsalzes, verwendet.

**28.** Verfahren nach mindestens einem der Ansprüche 7 bis 27, dadurch gekennzeichnet, daß man bei Atmosphärendruck, Überdruck oder Unterdruck arbeitet.

**29.** Verfahren nach einem der Ansprüche 7 bis 17, dadurch gekennzeichnet, daß man als Phasentransferkatalysator Mischungen von quartären Ammonium- oder Phosphoniumsalzen mit Polyethylenglykoldimethylethern einsetzt.

## Claims

1. An asymmetric halogenated benzophenone of the formula

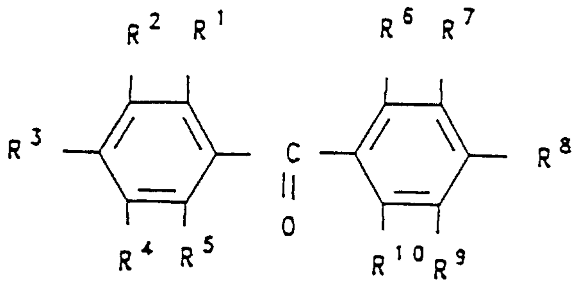

( 1 )

in which $R^1$ and $R^3$ are chlorine, $R^4$ is fluorine or $R^1$ is chlorine or fluorine, $R^3$ and $R^4$ are fluorine or $R^1$ is chlorine, $R^3$ is fluorine, $R^4$ is hydrogen and $R^2$, $R^5$, $R^6$, $R^7$, $R^8$, $R^9$ and $R^{10}$ are hydrogen or $R^1$ is chlorine, $R^3$, $R^4$ and $R^8$ are fluorine and $R^2$, $R^5$, $R^6$, $R^7$, $R^9$ and $R^{10}$ are hydrogen or $R^1$, $R^2$ and $R^3$ are chlorine, $R^4$ is chlorine or hydrogen, $R^8$ is fluorine and $R^5$, $R^6$, $R^7$, $R^9$ and $R^{10}$ are hydrogen or $R^1$, $R^3$ and $R^4$ are chlorine, $R^6$ is fluorine and $R^2$, $R^5$, $R^7$, $R^8$, $R^9$ and $R^{10}$ are hydrogen or $R^1$ and $R^6$ are chlorine, $R^2$, $R^3$ and $R^4$ are fluorine, $R^5$, $R^7$, $R^8$, $R^9$ and $R^{10}$ are hydrogen or $R^1$, $R^2$, $R^3$ and $R^4$ are fluorine, $R^7$ is chlorine or hydrogen, $R^8$ is chlorine, and $R^5$, $R^6$, $R^9$ and $R^{10}$ are hydrogen or $R^2$ and $R^3$ are chlorine, $R^8$ is fluorine and $R^1$, $R^4$, $R^5$, $R^6$, $R^7$, $R^9$ and $R^{10}$ are hydrogen or $R^1$ and $R^3$ are chlorine, $R^4$ is fluorine, $R^8$ is methyl and $R^2$, $R^5$, $R^6$ $R^7$, $R^9$ and $R^{10}$ are hydrogen or $R^3$ is fluorine, $R^8$ is methoxy, $R^1$, $R^2$, $R^4$, $R^5$, $R^6$, $R^7$, $R^9$ and $R^{10}$ are hydrogen or $R^2$ and $R^3$ are fluorine, $R^1$ and $R^4$ to $R^{10}$ are hydrogen.

2. A compound as claimed in claim 1 corresponding to the formula

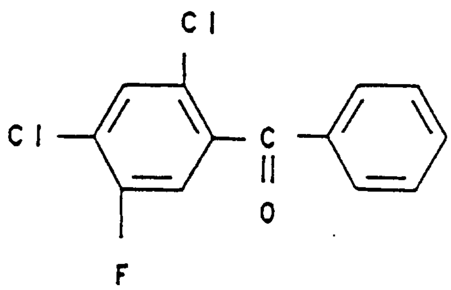

3. A compound as claimed in claim 1 corresponding to the formula

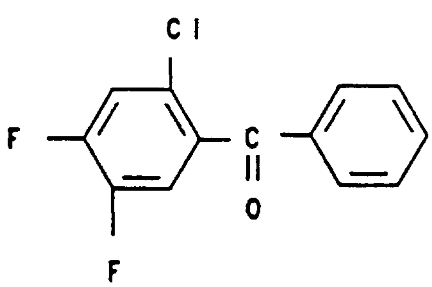

4. A compound as claimed in claim 1 corresponding to the formula

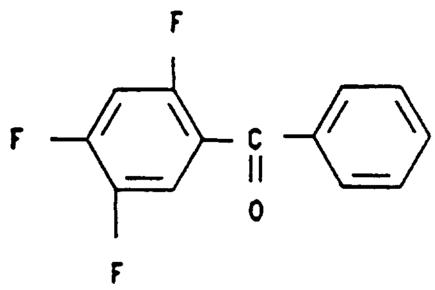

5. A compound as claimed in claim 1 corresponding to the formula

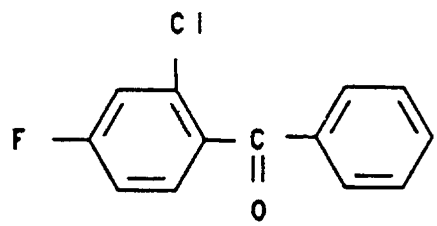

6. A compound as claimed in claim 1 corresponding to the formula

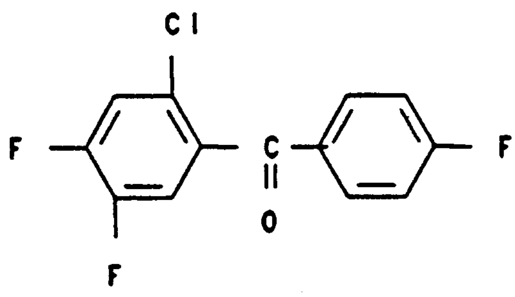

7. A process for the preparation of asymmetric halogenated benzophenones of the formula

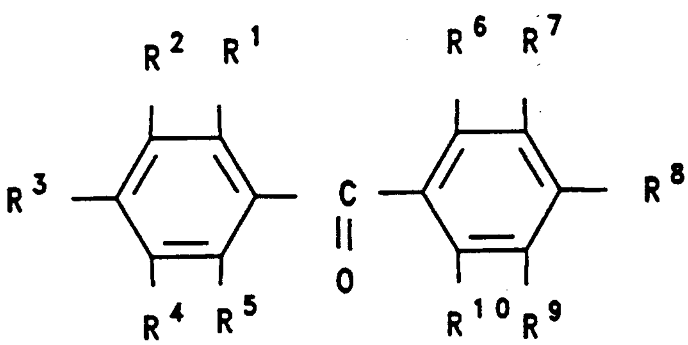 (1)

in which $R^1$ and $R^3$ are chlorine, $R^4$ is fluorine or $R^1$ is chlorine or fluorine, $R^3$ and $R^4$ are fluorine or $R^1$ is chlorine,

$R^3$ is fluorine, $R^4$ is hydrogen and $R^2$, $R^5$, $R^6$, $R^7$, $R^8$, $R^9$ and $R^{10}$ are hydrogen or $R^1$ is chlorine, $R^3$, $R^4$ and $R^8$ are fluorine and $R^2$, $R^5$, $R^6$, $R^7$, $R^9$ and $R^{10}$ are hydrogen or $R^1$, $R^2$ and $R^3$ are chlorine, $R^4$ is chlorine or hydrogen, $R^8$ is fluorine and $R^5$, $R^6$, $R^7$, $R^9$ and $R^{10}$ are hydrogen or $R^1$, $R^3$ and $R^4$ are chlorine, $R^6$ is fluorine and $R^2$, $R^5$, $R^7$, $R^8$, $R^9$ and $R^{10}$ are hydrogen or $R^1$ and $R^6$ are chlorine, $R^2$, $R^3$ and $R^4$ are fluorine, $R^5$, $R^7$, $R^8$, $R^9$ and $R^{10}$ are hydrogen or $R^1$, $R^2$, $R^3$ and $R^4$ are fluorine, $R^7$ is chlorine or hydrogen, $R^8$ is chlorine, and $R^5$, $R^6$, $R^9$ and $R^{10}$ are hydrogen or $R^2$ and $R^3$ are chlorine, $R^8$ is fluorine and $R^1$, $R^4$, $R^5$, $R^6$, $R^7$, $R^9$ and $R^{10}$ are hydrogen or $R^1$ and $R^3$ are chlorine, $R^4$ is fluorine, $R^8$ is methyl and $R^2$, $R^5$, $R^6$ $R^7$, $R^9$ and $R^{10}$ are hydrogen or $R^3$ is fluorine, $R^8$ is methoxy, $R^1$, $R^2$, $R^4$, $R^5$, $R^6$, $R^7$, $R^9$ and $R^{10}$ are hydrogen or $R^2$ and $R^3$ are fluorine, $R^1$ and $R^4$ to $R^{10}$ are hydrogen, which comprises acylating 1 mol of a halogenated benzene of the formula (2)

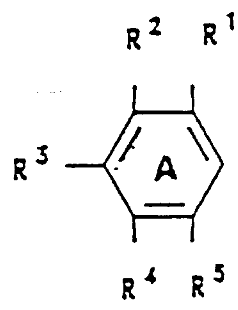

(2)

in which $R^1$, $R^2$, $R^3$, $R^4$ and $R^5$ are as defined above, with 1 to 5 mol of a benzoyl halide of the formula (3)

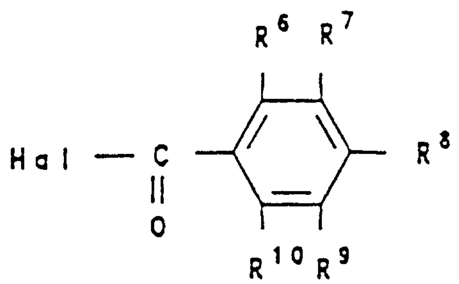

(3)

in which Hal is a fluorine, chlorine or bromine atom and $R^6$, $R^7$, $R^8$, $R^9$ and $R^{10}$ are as defined above, in the presence of an acylation catalyst, at temperatures from 0°C to 230°C, in the absence or in the presence of an organic solvent which is inert toward the reactants, and, if appropriate, reacting the resulting benzophenones of said formula (1), provided at least one of the substituents $R^1$ - $R^{10}$ is a chlorine atom, if appropriate after intermediate isolation thereof, with 1 to 2.5 mol of a potassium, rubidium or cesium fluoride, or mixtures thereof, per chlorine atom to be exchanged, at temperatures from 120 to 280°C, in the absence or in the presence of a phase transfer catalyst and in the absence or in the presence of a dipolar aprotic solvent.

8. The process as claimed in claim 7, wherein the acylation is carried out at temperatures from 70°C to 150°C.

9. The process as claimed in at least one of claims 7 and 8, wherein aluminum chloride, aluminum bromide, antimony(V) chloride, iron (III) chloride, iron (II) chloride, titanium(IV) chloride, boron trifluoride, tin(IV) chloride, bismuth(III) chloride, zinc chloride, mercury(II) chloride, hydrofluoric acid, sulfuric acid, polyphosphoric acid, p-toluenesulfonic acid or fluoroalkanesulfonic acids are used as acylation catalysts.

10. The process as claimed in at least one of claims 7 to 9, wherein aluminum trichloride is used as the acylation catalyst.

11. The process as claimed in at least one of claims 7 to 10, wherein the acylation is carried out in nitrobenzene, carbon disulfide, dichloromethane or 1,2-dichloroethane as the inert organic solvent.

12. The process as claimed in at least one of claims 7 to 11, wherein the acylation catalysts are used in amounts of 100 to 500 mol percent, based on benzoyl halide used.

13. The process as claimed in at least one of claims 7 to 12, wherein the acylation catalysts are used in amounts of

200 to 300 mol percent, based on benzoyl halide used.

**14.** The process as claimed in at least one of claims 7, 8 and 11, wherein metal-doped clay minerals are used as acylation catalysts.

**15.** The process as claimed in at least one of claims 7, 8, 11 and 14, wherein the metal-doped clay minerals are used in amounts of 2 to 100 percent by weight, based on benzoyl halide used.

**16.** The process as claimed in at least one of claims 7, 8, 11, 14 and 15, wherein the metal-doped clay minerals are used in amounts of 10 to 30 percent by weight, based on benzoyl halide used.

**17.** The process as claimed in at least one of claims 7, 8, 11, 14, 15 and 16, wherein the acylation reaction is carried out in the presence of metal-doped clay minerals at temperatures from 140°C to 220°C.

**18.** The process as claimed in at least one of claims 7 to 17, wherein quaternary ammonium or phosphonium compounds are used as the phase transfer catalyst.

**19.** The process as claimed in at least one of claims 7 to 18, wherein tetra-$C_1$-$C_{18}$-alkylammonium chlorides, bromides or fluorides, tetra-$C_1$-$C_{18}$-alkylphosphonium chlorides or bromides, tetraphenylphosphonium chloride or bromide or $((phenyl)_m(C_1$-$C_{18}$-$alkyl)_n)$-phosphonium chlorides or bromides, where $m = 1$ to 3, $n = 3$ to 1 and $m + n = 4$, are used as the phase transfer catalyst.

**20.** The process as claimed in at least one of claims 7 to 19, wherein the phase transfer catalyst is used in an amount of 0.01 to 50 mol percent, based on the halogenated benzophenone.

**21.** The process as claimed in at least one of claims 7 to 20, wherein the phase transfer catalyst is used in an amount of 0.5 to 10 mol percent, based on the halogenated benzophenone.

**22.** The process as claimed in at least one of claims 7 to 21, wherein the phase transfer catalyst is used in an amount of 1 to 5 mol percent, based on the halogenated benzophenone.

**23.** The process as claimed in at least one of claims 7 to 17, wherein oligoethylene or polyethylene glycol dimethyl ethers having 4 to 150 glycol units are used as the phase transfer catalyst.

**24.** The process as claimed in at least one of claims 7 to 17 and 23, wherein oligoethylene or polyethylene glycol dimethyl ethers having 3 to 25 glycol units are used as the phase transfer catalyst.

**25.** The process as claimed in at least one of claims 7 to 17, 23 and 24, wherein oligoethylene or polyethylene glycol dimethyl ethers are used as the phase transfer catalyst in amounts of 0.5 percent by weight to 200 percent by weight, based on the weight of the reaction salt used.

**26.** The process as claimed in at least one of claims 7 to 17 and 23 to 25, wherein the oligoethylene or polyethylene glycol ethers are used in amounts of 5 to 100 percent by weight, based on the weight of the fluoride salt used.

**27.** The process as claimed in at least one of claims 7 to 17 and 23 to 26, wherein the oligoethylene or polyethylene glycol ethers are used in amounts of 10 to 50 percent by weight, based on the weight of the fluoride salt used.

**28.** The process as claimed in at least one of claims 7 to 27, wherein the reaction is carried out under atmospheric pressure, excess pressure or reduced pressure.

**29.** The process as claimed in one of claims 7 to 17, wherein mixtures of quaternary ammonium or phosphonium salts with polyethylene glycol dimethyl ethers are used as the phase transfer catalyst.

**Revendications**

**1.** Benzophénones halogénées asymétriques de formule générale (1)

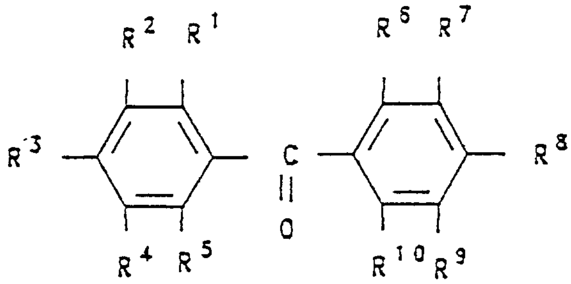

(1)

dans laquelle $R^1$, $R^3$ représentent des atomes de chlore, $R^4$ représente un atome de fluor ou $R^1$ représente un atome de chlore ou de fluor, $R^3$, $R^4$ représentent des atomes de fluor ou $R^1$ représente un atome de chlore, $R^3$ représente un atome de fluor, $R^4$ représente un atome d'hydrogène et $R^2$, $R^5$, $R^6$ ,$R^7$, $R^8$, $R^9$ et $R^{10}$ représentent des atomes d'hydrogène ou $R^1$ représente un atome de chlore, $R^3$, $R^4$ et $R^8$ représentent un atome de fluor et $R^2$, $R^5$, $R^6$, $R^7$, $R^9$ et $R^{10}$ représentent des atomes d'hydrogène ou $R^1$, $R^2$, $R^3$ représentent des atomes de chlore, $R^4$ représente un atome de chlore ou d'hydrogène, $R^8$ représente un atome de fluor et $R^5$, $R^6$, $R^7$, $R^9$ et $R^{10}$ représentent des atomes d'hydrogène ou $R^1$, $R^3$, $R^4$ représentent des atomes de chlore, $R^6$ un atome de fluor et $R^2$, $R^5$, $R^7$, $R^8$, $R^9$ et $R^{10}$ représentent des atomes d'hydrogène ou $R^1$, $R^6$ représentent des atomes de chlore, $R^2$, $R^3$, $R^4$ des atomes de fluor, $R^5$, $R^7$, $R^8$, $R^9$ et $R^{10}$ des atomes d'hydrogène ou $R^1$, $R^2$, $R^3$, $R^4$ des atomes de fluor, $R^7$ représente un atome de chlore ou d'hydrogène, $R^8$ représente un atome de chlore et $R^5$ ,$R^6$ ,$R^9$ et $R^{10}$ des atomes d'hydrogène ou $R^2$, $R^3$ représentent des atomes de chlore, $R^8$ représente un atome de fluor et $R^1$, $R^4$, $R^5$, $R^6$, $R^7$, $R^9$ et $R^{10}$ représentent des atomes d'hydrogène ou $R^1$, $R^3$ représentent des atomes de chlore, $R^4$ représente un atome de fluor, $R^8$ représente un groupe méthyle et $R^2$, $R^5$, $R^6$, $R^7$, $R^9$ et $R^{10}$ des atomes d'hydrogène ou $R^3$ représente un atome de fluor, $R^8$ représente un groupe méthoxy, $R^1$, $R^2$, $R^4$, $R^5$, $R^6$, $R^7$, $R^9$ et $R^{10}$ représentent des atomes d'hydrogène ou $R^2$, $R^3$ représentent des atomes de fluor, $R^1$ et $R^4$ à $R^{10}$ représentent des atomes d'hydrogène.

**2.** Composé selon la revendication 1 répondant à la formule

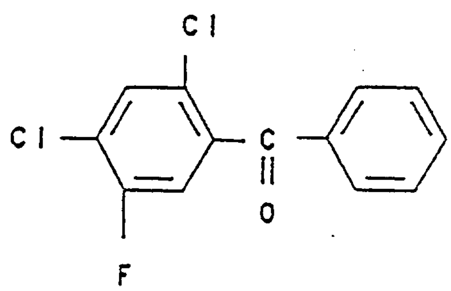

**3.** Composé selon la revendication 1 répondant à la formule

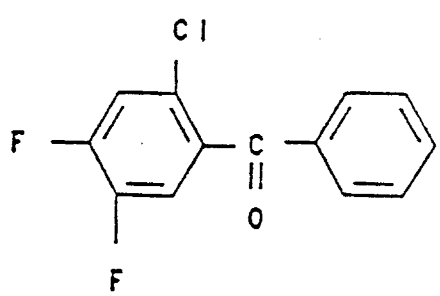

4. Composé selon la revendication 1 répondant à la formule

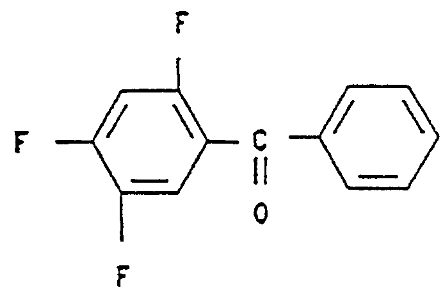

5. Composé selon la revendication 1 répondant à la formule

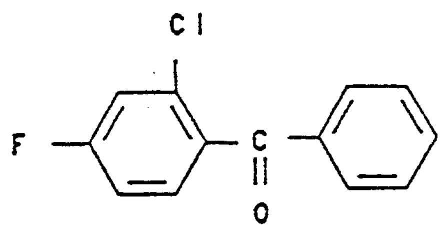

6. Composé selon la revendication 1 répondant à la formule

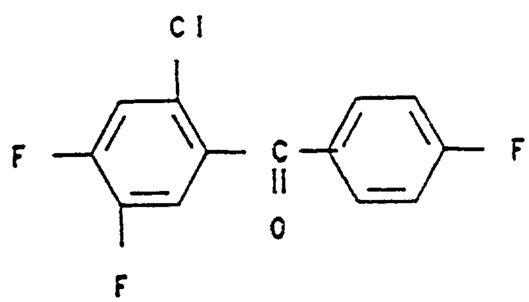

7. Procédé pour la préparation de benzophénones halogénées asymétriques de formule générale (1)

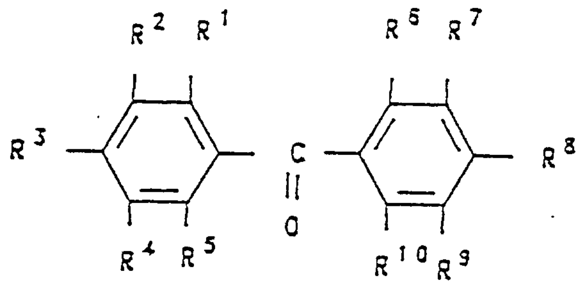

(1)

dans laquelle $R^1$, $R^3$ représentent des atomes de chlore, $R^4$ représente un atome de fluor ou $R^1$ représente un atome de chlore ou de fluor, $R^3$, $R^4$ représentent des atomes de fluor ou $R^1$ représente un atome de chlore, $R^3$ représente un atome de fluor, $R^4$ représente un atome d'hydrogène et $R^2$, $R^5$, $R^6$, $R^7$, $R^8$, $R^9$ et $R^{10}$ représentent des atomes d'hydrogène ou $R^1$ représente un atome de chlore, $R^3$, $R^4$ et $R^8$ représentent des atomes de fluor et $R^2$, $R^5$, $R^6$, $R^7$, $R^9$ et $R^{10}$ représentent des atomes d hydrogène ou $R^1$, $R^2$, $R^3$ représentent des atomes de chlore, $R^4$ représente un atome de chlore ou d'hydrogène, $R^8$ représente un atome de fluor et $R^5$, $R^6$, $R^7$, $R^9$ et $R^{10}$ représentent des atomes d'hydrogène ou $R^1$, $R^3$, $R^4$ représentent des atomes de chlore, $R^6$ représente un atome de fluor et $R^2$, $R^5$, $R^7$, $R^8$, $R^9$ et $R^{10}$ représentent des atomes d'hydrogène ou $R^1$, $R^6$ représentent des atomes de chlore, $R^2$, $R^3$, $R^4$ des atomes de fluor, $R^5$, $R^7$, $R^8$, $R^9$ et $R^{10}$ des atomes d'hydrogène ou $R^1$, $R^2$, $R^3$, $R^4$ des atomes de fluor, $R^7$ représente un atome de chlore ou d'hydrogène, $R^8$ représente un atome de chlore et $R^5$, $R^6$, $R^9$ et $R^{10}$ des atomes d hydrogène ou $R^2$, $R^3$ représentent des atomes de chlore, $R^8$ représente un atome de fluor et $R^1$, $R^4$, $R^5$, $R^6$, $R^7$, $R^9$ et $R^{10}$ représentent des atomes d'hydrogène ou $R^1$, $R^3$ représentent des atomes de chlore, $R^4$ représente un atome de fluor, $R^8$ représente un groupe méthyle et $R^2$, $R^5$, $R^6$, $R^7$, $R^9$ et $R^{10}$ des atomes d'hydrogène ou $R^3$ représente un atome de fluor, $R^8$ représente un groupe méthoxy, $R^1$, $R^2$, $R^4$, $R^5$, $R^6$, $R^7$, $R^9$ et $R^{10}$ représentent des atomes d'hydrogène ou $R^2$, $R^3$ représentent des atomes de fluor, $R^1$ et $R^4$ à $R^{10}$ représentent des atomes d'hydrogène, caractérisé en ce que l'on fait réagir 1 mole d'un benzène halogéné de formule générale (2)

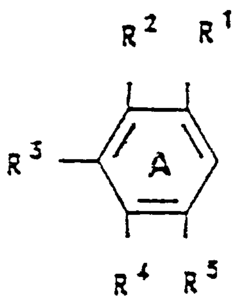

(2)

dans laquelle $R^1$, $R^2$, $R^3$, $R^4$, $R^5$ ont les significations données ci-dessus, avec 1 à 5 moles d'un halogénure de benzoyle de formule générale (3)

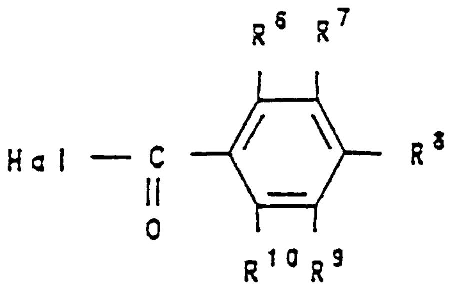

(3)

dans laquelle Hal représente un atome de fluor, de chlore ou de brome et $R^6$, $R^7$, $R^8$, $R^9$ et $R^{10}$ ont les significations données précédemment, en présence d'un catalyseur d'acylation, à une température de 0°C à 230 °C, en l'absence ou en présence d'un solvant organique inerte vis-à-vis des réactants et éventuellement, les benzophénones de formule (1) précitée ainsi obtenues, dans la mesure où au moins l'un des substituants $R^1$ à $R^{10}$ représente un atome de chlore, éventuellement après leur isolement intermédiaire, avec 1 à 2,5 moles d'un fluorure de potassium, de rubidium ou de caesium ou de leurs mélanges par atome de chlore à substituer, à une température de 120 à 280 °C, en l'absence ou en présence d'un catalyseur de transfert de phases et en l'absence ou en présence d'un solvant aprotique dipolaire.

8. Procédé selon la revendication 7, caractérisé en ce que l'on met en oeuvre l'acylation à une température de 70 à 150°C.

9. Procédé selon au moins l'une des revendications 7 et 8, caractérisé en ce que l'on utilise en tant que catalyseur d'acylation du chlorure d'aluminium, du bromure d'aluminium, du chlorure d'antimoine(V), du chlorure de fer(III), du chlorure de fer(II), du chlorure de titane(IV), du trifluorure de bore, du chlorure d'étain(IV), du chlorure de bismuth

(III), du chlorure de zinc, du chlorure de mercure(II), l'acide fluorhydrique, l'acide sulfurique, l'acide polyphosphorique, l'acide p-toluènesulfonique ou des acides fluoralcanesulfoniques.

**10.** Procédé selon au moins l'une des revendications 7 à 9, caractérisé en ce que l'on utilise en tant que catalyseur d'acylation du trichlorure d'aluminium.

**11.** Procédé selon au moins l'une des revendications 7 à 10, caractérisé en ce que l'on acyle dans du nitrobenzène, du sulfure de carbone, du dichlorométhane ou du 1,2-dichloréthane en tant que solvant organique inerte.

**12.** Procédé selon au moins l'une des revendications 7 à 11, caractérisé en ce que l'on utilise les catalyseurs d'acylation dans des quantités de 100 à 500 % en moles par rapport à l'halogénure de benzoyle utilisé.

**13.** Procédé selon au moins l'une des revendications 7 à 12, caractérisé en ce que l'on utilise les catalyseurs d'acylation dans des quantités de 200 à 300 % en moles par rapport à l'halogénure de benzoyle utilisé.

**14.** Procédé selon au moins l'une des revendications 7, 8 et 11, caractérisé en ce que l'on utilise en tant que catalyseur d'acylation des minerais argileux dopés par des métaux.

**15.** Procédé selon au moins l'une des revendications 7, 8, 11 et 14, caractérisé en ce que l'on utilise les minerais argileux dopés par des métaux dans des quantités de 2 à 100 % en masse par rapport à l'halogénure de benzoyle utilisé.

**16.** Procédé selon au moins l'une des revendications 7, 8, 11, 14 et 15, caractérisé en ce que l'on utilise des minerais argileux dopés par des métaux dans des quantités de 10 à 30 % en masse par rapport à l'halogénure de benzoyle utilisé.

**17.** Procédé selon au moins l'une des revendications 7, 8, 11, 14, 15 et 16, caractérisé en ce que l'on met en oeuvre la réaction d'acylation à une température de 140 à 220 °C en présence de minerais argileux dopés par des métaux.

**18.** Procédé selon au moins l'une des revendications 7 à 17, caractérisé en ce que l'on utilise en tant que catalyseur de transfert de phases des composés d'ammonium ou de phosphonium quaternaire.

**19.** Procédé selon au moins l'une des revendications 7 à 18, caractérisé en ce que l'on utilise en tant que catalyseur de transfert de phases des chlorures, des bromures ou des fluorures de tétra(alkyl en $C_1$-$C_{18}$)-ammonium, des bromures ou des chlorures de tétra(alkyl en $C_1$-$C_{18}$)phosphonium, le chlorure ou le bromure de tétraphénylphosphonium, des bromures ou des chlorures de ((phényl)$_m$(alkyl en $C_1$-$C_{18}$))$_n$)-phosphonium, m = 1 à 3, n = 3 à 1 et m+n = 4 .

**20.** Procédé selon au moins l'une des revendications 7 à 19, caractérisé en ce que l'on utilise le catalyseur de transfert de phases dans une quantité de 0,01 à 50 % en moles par rapport a la benzophénone halogénée.

**21.** Procédé selon au moins l'une des revendications 7 à 20, caractérisé en ce que l'on utilise le catalyseur de transfert de phases dans une quantité de 0,5 à 10 % en moles par rapport à la benzophénone halogénée.

**22.** Procédé selon au moins l'une des revendications 7 à 21, caractérisé en ce que l'on utilise le catalyseur de transfert de phases dans une quantité de 1 à 5 % en moles par rapport à la benzophénone halogénée.

**23.** Procédé selon au moins l'une des revendications 7 à 17, caractérisé en ce que l'on utilise comme catalyseur de transfert de phases des oligo- ou polyéthylèneglycoldiméthyléthers avec 4 à 150 motifs glycol.

**24.** Procédé selon au moins l'une des revendications 7 à 17 et 23, caractérisé en ce que l'on utilise en tant que catalyseur de transfert de phases des oligo- ou polyéthylèneglycoldiméthyléthers avec 3 à 25 motifs glycol.

**25.** Procédé selon au moins l'une des revendications 7 à 17, 23 et 24, caractérisé en ce que l'on utilise en tant que catalyseur de transfert de phases des oligo- ou polyéthylèneglycoldiméthyléthers dans des quantités de 0,5 % en masse à 200 % en masse par rapport à la masse du sel réactionnel utilisé.

**26.** Procédé selon au moins l'une des revendications 7 à 17 et 23 à 25, caractérisé en ce que l'on utilise les oligo- ou

les polyéthylèneglycoléthers dans des quantités de 5 à 100 % en masse par rapport à la masse du sel fluorure utilisé.

**27.** Procédé selon au moins l'une des revendications 7 à 17 et 23 à 26, caractérisé en ce que l'on utilise les oligo- ou les polyéthylèneglycoléthers en quantités de 10 à 50 % en masse par rapport à la masse du sel fluorure utilisé.

**28.** Procédé selon au moins l'une des revendications 7 à 27, caractérisé en ce que l'on opère à la pression atmosphérique, sous une surpression ou sous un défaut de pression.

**29.** Procédé selon l'une des revendications 7 à 17, caractérisé en ce que l'on utilise comme catalyseur de transfert de phases des mélanges de sels d'ammonium ou de phosphonium quaternaires avec des polyéthylèneglycoldiméthyléthers.